(19) Europäisches Patentamt
European Patent Office
Office européen des brevets

(11) EP 3 020 398 A1

(12) EUROPEAN PATENT APPLICATION

(43) Date of publication:
**18.05.2016 Bulletin 2016/20**

(51) Int Cl.:
***A61K 31/202*** *(2006.01)* ***A61K 9/70*** *(2006.01)*
***A61P 17/02*** *(2006.01)*

(21) Application number: **14193497.6**

(22) Date of filing: **17.11.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NITTO DENKO CORPORATION**
**Ibaraki-shi, Osaka 567-8680 (JP)**

(72) Inventor: **De Visscher, Geofrey**
**1071 Chexbres (CH)**

(74) Representative: **Hoffmann Eitle**
**Patent- und Rechtsanwälte PartmbB**
**Arabellastraße 30**
**81925 München (DE)**

# (54) Compounds and formulations for reducing scarring

(57) The present invention relates to the reduction of scarring in wounds. More particularly, it relates to compounds according to Formula 1 for use in methods of reducing scarring, to formulations which incorporate these compounds and to a method of treating wounds using such compounds and formulations so as to reduce scarring:

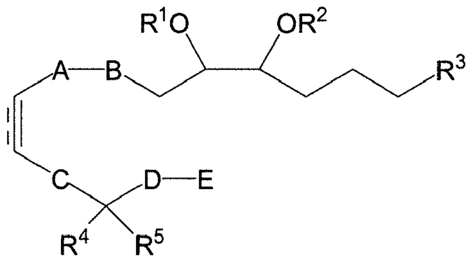

Formula 1

wherein

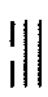 is a double bond or triple bond, preferably a double bond;

A, B and C are the same or different and selected from $CH_2CH_2$, *cis*-CH=CH, *trans*-CH=CH and C≡C;

$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;

$R^3$ is $CO_2R^{31}$, $CONR^{32}R^{33}$, $CH_2OR^{34}$, $CH_2NR^{35}R^{36}$, $CH_2N_3$, $CH_2$-X, $CH_2NO_2$, $CH_2SR^{37}$, $COSR^{38}$, or 2,3,4,5-tetrazol-1-yl, wherein:

$R^{31}$ is H or $C_{1-6}$ alkyl;

$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;

$R^{34}$ is H, $C_{1-6}$ alkyl, or $C_{2-6}$ acyl;

$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$

EP 3 020 398 A1

cycloalkenyl, $C_{6-12}$ aralkyl, $C_{5-10}$ aryl, OH, or $C_{1-6}$ alkoxy;
X is F, Cl, Br or I;
$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;

$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;
D is $(CR^6R^7)_q$ or $(CR^6R^7)_qO$, where q is 0-6; and $R^6$ and $R^7$ are the same or different and selected from H or $C_{1-6}$ alkyl;
E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

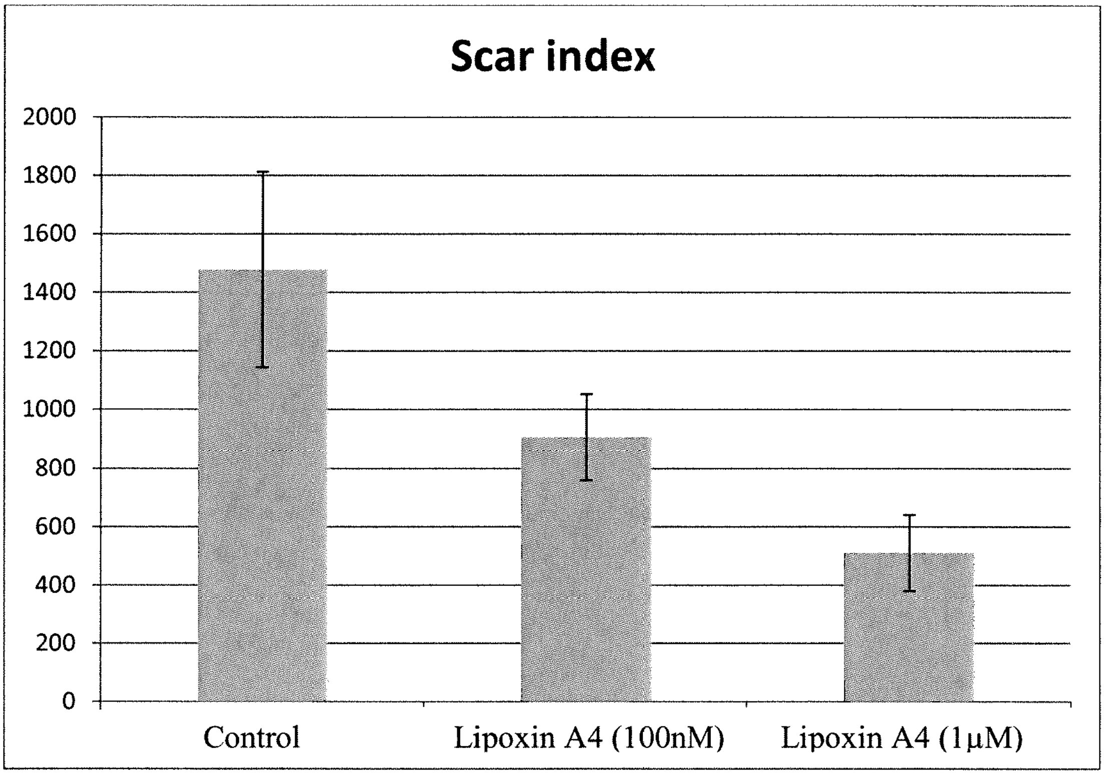


FIG. 4

## Description

### 1. Field of the Invention

The present invention relates to the reduction of scarring in wounds. More particularly, the invention relates to compounds that reduce the formation of scars, to formulations and devices which incorporate these compounds and to a method of treating wounds using such compounds and formulations so as to reduce scarring.

### 2. Background of the Invention

#### *2.1 Pathophysiology of scar tissue formation*

The healing of wounds (e.g. dermal wounds) is a complex process involving the collaboration of several tissues and cell lineages. This healing is mediated by local cells such as dermal fibroblasts and keratinocytes. However, cells from the immune system also play a crucial role in regulating the healing by releasing soluble factors influencing nearby cells but also by modulating the extracellular matrix (ECM). Consequently re-epithelialization is crucial as is the regeneration of an 'adequate' ECM. In terms of scarring this repaired ECM should not be bulky, provide adequate strength and also be organised comparable to the normal skin tissue. In this last part collagens play an important role.

The primary objective of the healing process is to fill the gap created by tissue destruction and restore the structural continuity of the injured part. The gap is normally filled with a fibrous tissue otherwise known as scar tissue. With the exception of very minor and superficial lesions, every wound (e.g. after accident, disease, or surgery) results in some degree of scarring. The biological repair process differs little from one kind of tissue to another and is generally the same for all acute wounds, open or closed, regardless of how they are caused. The normal wound healing process is a cascade of overlapping events that are commonly divided into four phases: the coagulation phase, the inflammatory phase, the proliferative phase and the remodelling phase.

The initial coagulation phase provides haemostasis by depositing a fibrin clot which also comprises platelets. This clot will provide a first extracellular matrix aiding the cells of the later phases to move in and repair the damage. When tissue is first wounded, a vascular spasm causes blood vessels in the vicinity of the wound to constrict thereby minimising blood loss. Exposure of blood to proteins in the tissue (e.g. thromboplastin) initiates changes to blood platelets and the soluble plasma protein fibrinogen. Platelets immediately form a plug at the site of injury (primary haemostasis) while soluble fibrinogen is converted through a complex cascade to insoluble fibrin strands which strengthen the platelet plug (secondary haemostasis). Fibrin and fibronectin cross-link together to form a plug that provides the main structural support for the wound until collagen is deposited. The platelets will provide an initial surge of factor that will initiate and steer the inflammatory reaction.

The major function of the inflammatory phase is to remove damaged tissue and foreign matter from the wound and fight infectious agents, thus preparing the wound environment for healing. Vasoconstriction lasts five to ten minutes and is followed by vasodilatation, a widening of blood vessels, which peaks at about 20 minutes post-wounding. Vasodilatation is accompanied by small vessel permeability in the region of the wound. The resulting oedema in the area of the injury is the source of the familiar pain and swelling which occurs early after injury. The increased porosity of blood vessels facilitates the entry of circulating white blood cells such as polymorphonuclear neutrophils (PMN) and monocytes from the bloodstream into the wound site. PMN initially appear in the wound shortly after injury and their numbers increase steadily, peaking at about 24 to 48 h. They function as a first responder and engulf and degrade any bacteria in the wound in a process called phagocytosis. Monocytes/macrophages are also attracted to the wound site by growth factors released by platelets and other cells and, once in the wound site, mature into macrophages where they supersede PMN as the major wound phagocyte. These macrophages also release growth factors such as platelet-derived growth factor

(PDGF) and transforming growth factor beta (TGF-β). These growth factors attract and activate fibroblasts from surrounding tissue into the wound where they then proliferate.

The arrival of fibroblasts (about 2 to 5 days post wounding) marks the beginning of the proliferative phase of wound healing where the focus moves to the building of new tissue to fill the wound space. The proliferative phase is characterized by the formation of granulation tissue in the wound that serves as the foundation for scar tissue development. Granulation tissue functions as rudimentary tissue, and begins to appear in the wound already during the inflammatory phase and continues growing until the wound bed is covered. Granulation tissue consists of a combination of cellular elements, including fibroblasts and inflammatory cells, along with new capillaries embedded in a loose extracellular matrix of collagen, fibronectin and hyaluronan. Fibroblasts are the primary synthetic element in the repair process and are responsible for the production of the majority of structural proteins used during tissue reconstruction. In particular, fibroblasts produce large quantities of collagen, a family of triple-chain glycoproteins, which form the main constituent of the extracellular wound matrix and which are ultimately responsible for imparting tensile strength to the scar. Even as fibroblasts are producing new collagen, collagenases and other factors degrade it. Shortly after wounding, the rate

of synthesis of collagen exceeds its degradation so collagen levels in the wound rise, but later production and degradation become equal so there is no net collagen gain. The fibroblasts also secrete growth factors that attract epithelial cells to the wound site. Angiogenesis, or neovascularization, occurs concurrently with fibroblast proliferation and the formation of capillaries in the granulation tissue allows vital oxygen and nutrients to be delivered to the wound site. The final component of the proliferative phase is epithelialization which is the regeneration, migration, proliferation and differentiation of epithelial cells at the wound's edge to form a new surface area similar to that destroyed by the injury. By the end of the proliferative stage white blood cells leave the wound site, oedema diminishes, and the wound begins to blanch as the small blood vessels become occluded by thrombosis and degenerate.

The fourth phase of wound healing is the remodelling phase which begins about three weeks post wound and can continue for six months or longer. The remodelling phase is generally said to begin when the levels of collagen production and collagen degradation equalize. During this phase, type III collagen, which is prevalent during the proliferative phase, is replaced by type I collagen thus forming the final scar tissue. The collagen fibrils, which previously were randomly oriented, align in the direction of mechanical tension, providing further mechanical strength to the wound. Upon completion of the entire process, the skin regains its chemical and physical barrier functions. Clinically, the scar becomes avascular and the scar tissue may achieve up to 70-80% of tensile strength of normal tissue by the end of three months. A review of the processes involved in wound healing was published by Singer and Clark (1999).

### *2.2 Problems with scar tissue*

Scar tissue formation is a natural part of the healing process after wound injury, and while it is often desirable to increase the rate of healing for acute and chronic wounds, in some instances the regulation of scar formation is of primary importance and the rate of wound healing is a secondary consideration. In some cases, an exaggerated healing response can result in the production of copious amounts of scar tissue (e.g. hypertrophic and keloid scarring). Excessive scarring can be a major medical problem, often resulting in loss of function, restriction of tissue movement and/or growth. For instance, sweat glands or hair follicles do not exist in scar tissue. This can impair the regulation of body temperature, particularly around the scar area. Moreover, scars can reduce the flexibility of the tissue in which they form. For instance, scarring can reduce movement of the skin on the neck, shoulders and joints such as the jaws, elbows and knee caps. Scarring can also have adverse aesthetic effects (e.g. discoloration) which in some cases may lead to adverse psychological effects. Social, emotional and psychological effects of scars are especially notable on individuals with visible scars.

Where scars have already formed, existing therapeutic strategies for their treatment include pressure therapy, silicone gel sheeting, intra-lesional TAC, cryosurgery, radiation, laser therapy, INF, 5-FU and surgical excision. However, these therapeutic strategies have the disadvantage of only treating scars that have already formed. It would be better to reduce the amount of scarring that occurs in the first place.

## 3. Summary of the Invention

The present invention relates to the reduction of scarring in wounds. More particularly, the invention relates to compounds that reduce the formation of scars, to formulations and devices which incorporate these compounds and to a method of treating wounds using such compounds and formulations so as to reduce scarring.

Compounds for use in methods of reducing scarring in wounds according to the present invention include those according to Formula 1:

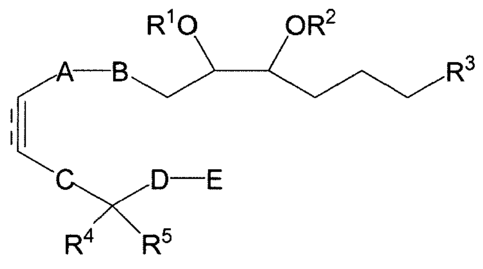

Formula 1

wherein

is a double bond or triple bond, preferably a double bond;

A, B and C are the same or different and selected from $CH_2CH_2$, *cis*-CH=CH, *trans*-CH=CH and C≡C;
$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;
$R^3$ is $CO_2R^{31}$, $CONR^{32}R^{33}$, $CH_2OR^{34}$, $CH_2NR^{35}R^{36}$, $CH_2N_3$, $CH_2$-X, $CH_2NO_2$, $CH_2SR^{37}$, $COSR^{38}$, or 2,3,4,5-tetrazol-1-yl, wherein:
$R^{31}$ is H or $C_{1-6}$ alkyl;
$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
$R^{34}$ is H, $C_{1-6}$ alkyl, or $C_{2-6}$ acyl;
$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
X is F, Cl, Br or I;
$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;
$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;
D is $(CR^6R^7)_q$ or $(CR^6R^7)_qO$, where q is 0-6; and $R^6$ and $R^7$ are the same or different and selected from H or $C_{1-6}$ alkyl;
E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

The compound according to Formula 1 can be formulated as a topical formulation and the topical formulation may comprise more than one compound according to Formula 1.

Surprisingly, it has been found that when compounds according to Formula 1 or topical formulations containing these compounds are applied to a wound the amount of scar tissue formed after wound healing is significantly reduced. Even more surprisingly, the scar tissue that does form exhibits characteristics more closely associated with normal functioning tissue. Reduced scar formation correlated to a change in the extracellular matrix of the scar, particularly decreased collagen organization as measured in the wound cross-section. The large stress fibers that normally develop upon scarring were less pronounced in wounds treated with compounds according to the present invention. Thus, the invention also relates to compounds according to Formula 1, and compositions containing such compounds, for use in methods of reducing scarring in wounds by reducing collagen organization in a healing wound.

The wound to be treated can be an acute wound or a chronic wound. The wound may be an open wound or a closed wound. When the compounds and the formulations of the invention are applied to a wound, a reduction in scar formation can be observed. Thus, the compounds and the formulations of the invention can be used to reduce the formation of scars such as normal scars, hypertrophic scars, keloid scars, atrophic scars or striae (stretch marks).

The present invention further relates to an adhesive patch or suture comprising at least one compound according to Formula 1. Adhesive patches and sutures are a particularly effective means of delivering the compounds of Formula 1 to a wound. The present invention also relates to other topical administration forms of at least one compound according to Formula 1. Such other topical administration forms include for instance administration via a vaseline or oily substance coated mesh, which is an administration form often used in open wound treatment and larger defects. Another topical administration form according to the present invention is a dressing with an absorbing patch in the middle (standard plaster type) where the compound according to Formula 1 is either included in the mass or administered on the surface in a bioresorbable matrix. The mass implied can be but is not limited to cotton gauze, viscose gauze, non-woven absorbing material, absorbing materials with gelling agents such as alginate, pectin or CMC, absorbing materials coated with non-adherent film, anti-bacterial absorbing materials, hydrogels, silicon gels, polyurethane sponges, polyurethane granulate, polyacrylate absorbers, polyacrylamide derivatives, hydrocolloids, lipocolloids, PVA-foam, cellulose, polyurethane gel. Yet another topical administration form is envisaged for the treatment of wounds inflicted by wet shaving: the compound of Formula 1 may be included in the razor cartridge to permit continuous application of the compound during shaving. Alternatively, the compound may be included in aftershave compositions, lotions or creams.

Other objects, effects and advantages of the present invention will be apparent from the following description.

## 4. Brief Description of the Figures

**Figure 1** is a schematic representation of an example of the procedure in the animal model for scar analysis.

**Figure 2** shows a H&E image and the corresponding Sirius red image illustrating how measurements are taken for

scar size analysis.

**Figure 3** shows the results of the quantitative scar index analysis. Wounds treated with Lipoxin A4 exhibit significantly lower scarring (49%) than wounds treated with Control (n=5; p<0.008).

**Figure 4** shows the results of the quantitative scar index analysis for treatment with different doses of Lipoxin A4.

**Figure 5** shows the average collagen content and organisation for wounds treated with Lipoxin A4 and wounds treated with vehicle.

## 5. Detailed Description of the Preferred Embodiments

### *5.1 Definitions*

A "wound" as used in the present invention is to be understood as the disruption of integrity of the skin, subcutaneous tissue, connective tissue or muscular tissue. In this respect skin means the membranous protective covering of the body that includes the epidermis and dermis; subcutaneous tissue means the irregular layer of loose connective tissue immediately deep to the skin and superficial to the deep fascia; connective tissue means the supporting or framework tissue of the body including areolar or loose tissue, adipose tissue, dense, regular or irregular, white fibrous tissue, elastic tissue, mucous tissue, lymphoid tissue, cartilage, and bone; and muscular tissue includes skeletal, cardiac and smooth tissue.

Wounds may comprise minor cuts or abrasions; complicated/full thickness wounds; traumatic wounds such as e.g. abrasions, lacerations; surgical wounds; post-surgical incisions; or chronic/non-healing wounds like pressure sores; diabetic ulcers; injuries to connective tissue like bone or cartilage; burn injuries; and scars (e.g. keloid scars, contracture scars, hypertrophic scars and acne scars). The wound may be an open wound or a closed wound. A 'closed' wound in the sense of the present invention means a wound that was open at one point (e.g. a surgical incision or an accidental cut) and that has been purposefully closed by means of a suture, staple, tape, surgical adhesive and the like.

A "scar" in the sense of the present invention means normal scars, hypertrophic scars, keloid scars, contracture scars, atrophic scars and striae. Symptoms of scars include skin discolorations (including redness, changes in pigmentation, or other discolorations e.g. from blanching), erythema, dry, flaky, or itchy skin, raised area above the surrounding skin, keloid formation, hypertrophy, scar pain, decreased vascularisation of the scar and/or surrounding tissue, reduced pliability, and poor aesthetic appearance (including quality and texture of the scar tissue). Thus, for the purpose of the present invention, the reduction of scarring can also be considered as the treatment (prevention or amelioration) of these symptoms.

"Topical administration" in the sense of the present invention means administration to a definite surface, e.g. directly to the wound surface, or if the wound has been closed, to the area around the closure.

### *5.2 Active Compounds*

The compounds suitable for practising the invention are those according to Formula 1:

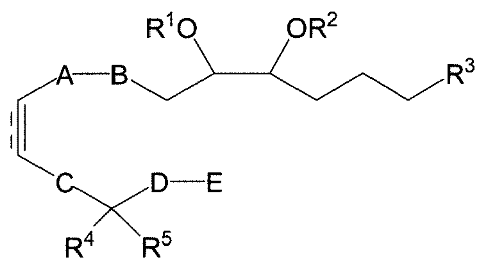

Formula 1

wherein

is a double bond or triple bond, preferably a double bond;
A, B and C are the same or different and selected from $CH_2CH_2$, *cis*-CH=CH, *trans*-CH=CH and C≡C;

$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;

$R^3$ is $CO_2R^{31}$, $CONR^{32}R^{33}$, $CH_2OR^{34}$, $CH_2NR^{35}R^{36}$, $CH_2N_3$, $CH_2$-X, $CH_2NO_2$, $CH_2SR^{37}$, $COSR^{38}$, or 2,3,4,5-tetrazol-1-yl, wherein:

$R^{31}$ is H or $C_{1-6}$ alkyl;

$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;

$R^{34}$ is H, $C_{1-6}$ alkyl, or $C_{2-6}$ acyl;

$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;

X is F, Cl, Br or I;

$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;

$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;

D is $(CR^6R^7)q$ or $(CR^6R^7)qO$, where q is 0-6; and $R^6$ and $R^7$ are the same or different and selected from H or $C_{1-6}$ alkyl;

E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

Preferred compounds are those having Formula 2 where $R^1$-$R^5$, D and E are as defined above for Formula 1:

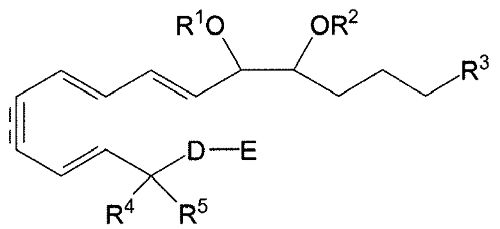

Formula 2

Compounds that are suitable for use in the invention include those having Formula 2 where $R^1$ and $R^2$ are H. Further compounds include those where $R^3$ is COOH. Further compounds include those where $R^4$ is H or $CH_3$. Further compounds include those where $R^5$ is OH. Even further compounds include those where $R^1$ and $R^2$ are H, and $R^3$ is COOH; or $R^1$ and $R^2$ are H, and $R^4$ is H or $CH_3$; or $R^1$ and $R^2$ are H, and $R^5$ is OH; or $R^3$ is COOH, and $R^4$ is H or $CH_3$; or $R^3$ is COOH, and $R^5$ is OH; or $R^4$ is H or $CH_3$, and $R^5$ is OH. Even further compounds include those where $R^1$ and $R^2$ are H, $R^3$ is COOH, and $R^4$ is H or $CH_3$; or $R^1$ and $R^2$ are H, $R^3$ is COOH, and $R^5$ is OH; or $R^3$ is COOH, $R^4$ is H or $CH_3$, and $R^5$ is OH; or $R^1$ and $R^2$ are H, $R^3$ is COOH, $R^4$ is H or $CH_3$, and $R^5$ is OH.

Preferred compounds are those having one of the following Formulae 2-1 to 2-5 where $R^1$-$R^5$, D and E are as defined above for Formula 1:

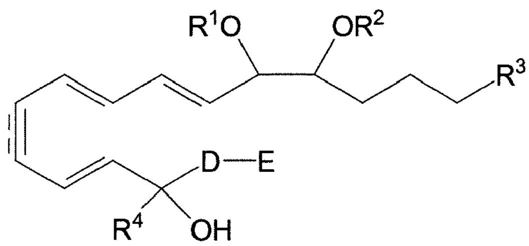

Formula 2-1

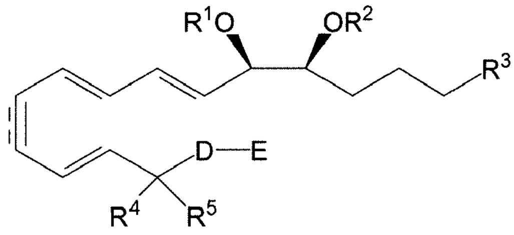

Formula 2-2

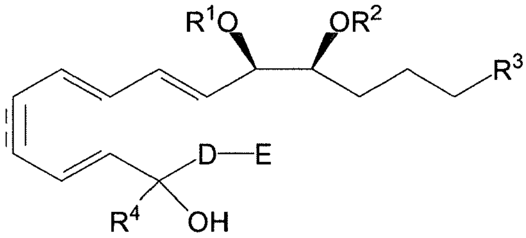

Formula 2-3

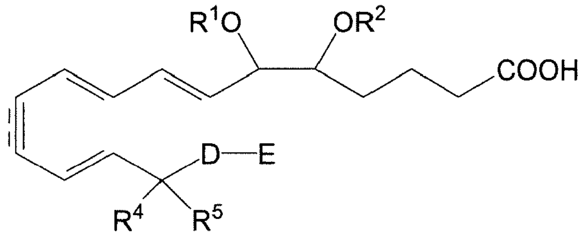

Formula 2-4

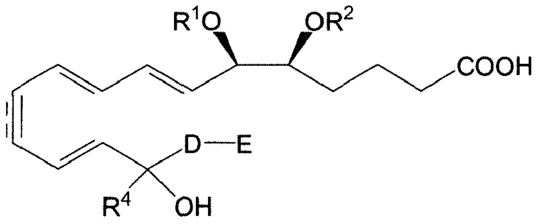

Formula 2-5

Compounds that are suitable for use in the invention include those having formula 2-1 to 2-5 and where $R^1$ and $R^2$ are H. Further compounds include those where $R^3$ is COOH. Further compounds include those where $R^4$ is H or $CH_3$. Further compounds include those where $R^5$ is OH. Even further compounds include those where $R^1$ and $R^2$ are H, and $R^3$ is COOH; or $R^1$ and $R^2$ are H, and $R^4$ is H or $CH_3$; or $R^1$ and $R^2$ are H, and $R^5$ is OH; or $R^3$ is COOH, and $R^4$ is H or $CH_3$; or $R^3$ is COOH, and $R^5$ is OH; or $R^4$ is H or $CH_3$, and $R^5$ is OH. Even further compounds include those where $R^1$ and $R^2$ are H, $R^3$ is COOH, and $R^4$ is H or $CH_3$; or $R^1$ and $R^2$ are H, $R^3$ is COOH, and $R^5$ is OH; or $R^3$ is COOH, $R^4$ is H or $CH_3$, and $R^5$ is OH; or $R^1$ and $R^2$ are H, $R^3$ is COOH, $R^4$ is H or $CH_3$, and $R^5$ is OH.

For any of the above-mentioned compounds according to Formula 1, Formula 2, Formula 2-1, Formula 2-2, Formula 2-3, Formula 2-4, or Formula 2-5, D is $(CR^6R^7)_q$ or $(CR^6R^7)qO$, where q is 0-6; and $R^6$ and $R^7$ are the same or different and selected from H or $C_{1-6}$ alkyl. Preferably D is $-CH_2-$, $-(CH_2)_2-$, $-(CH_2)_3-$, $-(CH_2)_4-$, $-(CH_2)_5-$, $-(CH_2)_6-$, $-CH_2O-$, $-(CH_2)_2O-$, $-(CH_2)_3O-$, $-(CH_2)_4O-$, $-(CH_2)_5O-$ or - $(CH_2)_6O-$.

For any of the above-mentioned compounds according to Formula 1, Formula 2, Formula 2-1, Formula 2-2, Formula 2-3, Formula 2-4, or Formula 2-5, together with or independently of the preferred definition for D above, E is $CH_3$, or a cyclohexyl or phenyl ring optionally substituted with one or more substituents selected from $C_{1-6}$ alkyl, halogen,

trihalomethyl, $C_{2-6}$ acyl, hydroxy, thiol, or amino group. If substituted, the cyclohexyl or phenyl ring is preferably substituted with 1, 2, 3 or 4 substituents which may be the same or different. Halogen is a preferred substituent, with F being most preferred.

In a preferred embodiment, for any of the above-mentioned compounds according to Formula 1, Formula 2, Formula 2-1, Formula 2-2, Formula 2-3, Formula 2-4, or Formula 2-5, D is $-(CH_2)_4-$ and E is $CH_3$; or D is $-CH_2O-$ and E is a phenyl ring optionally substituted with F, preferably substituted with F at the para position; or q=0 and E is cyclohexyl.

For all of the above-mentioned compounds, preferably represents a double bond.

Preferred compounds for use in the present invention include Lipoxin A4 ($LXA_4$), 15-epi-$LXA_4$, 16-phenoxy-$LXA_4$, 15-epi-16-phenoxy-$LXA_4$, 15(R/S)-methyl-$LXA_4$, 15(R/S)-16-phenoxy-11,12-acetylenic-$LXA_4$ or any pharmaceutically acceptable salt or solvent thereof.

### *5.3 Pharmaceutically acceptable salts and solvates*

Unless specified otherwise, it is to be understood that any disclosure herein with respect to the compounds of Formula (1), or the preferred compounds according to Formula 2 or Formulae 2-1 to 2-5 a disclosed herein, also extends to the stereoisomers, tautomers, pharmaceutically acceptable salts, polymorphs, and solvates thereof.

"Pharmaceutically acceptable salts", as used herein, are salts that retain the desired biological activity of the parent compound and do not impart undesired toxicological effects. Pharmaceutically acceptable salt forms include various crystalline polymorphs as well as the amorphous form of the different salts. The pharmaceutically acceptable salts can be formed with metal or organic counterions and include, but are not limited to, alkali metal salts such as sodium or potassium; alkaline earth metal salts such as magnesium or calcium; and ammonium or tetraalkyl ammonium salts. The salts can be organic or inorganic in nature. Representative salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate.

"Solvates", as used herein, are addition complexes in which the compound is combined with an acceptable co-solvent in some fixed proportion. Co-solvents include, but are not limited to, ethyl acetate, lauryl lactate, myristyl lactate, cetyl lactate, isopropyl myristate, methanol, ethanol, 1-propanol, isopropanol, 1-butanol, isobutanol, tert-butanol, acetone, methyl ethyl ketone, acetonitrile, benzene, toluene, xylene(s), ethylene glycol, dichloromethane, 1,2-dichloroethane, N-methylformamide, N,N-dimethylformamide, N-methylacetamide, pyridine, dioxane, and diethyl ether. The term 'hydrate' is employed when the co-solvent is water.

### *5.4 Formulation types*

The compounds of Formula 1, Formula 2 or Formulae 2-1 to 2-5 are preferably formulated as a composition suitable for topical administration such as a lotion, cream, gel, ointment, paste, emulsion, foam, mousse, solution, spray, dispersion, aerosol, alginates, hydrogels, and hydrocolloids sticks, bars, and films. Reference is made to *"Remington: The Science and Practice of Pharmacy, 21st Ed., Chapter 39"* and *"Ansel's Pharmaceutical Dosage Forms and Drug Delivery Systems, 9th Ed., Section IV-10 and Section VI"* for further information regarding topical formulations. Preferred topical formulations are lotions, creams, gels, and ointments. The compounds of Formula 1, Formula 2 or Formulae 2-1 to 2-5 are also effective when used in adhesive compositions such as a medical or surgical glue. The compounds of Formula 1, Formula 2 or Formulae 2-1 to 2-5 are also effective when administered to a wound by injection and thus can be formulated as an injection solution.

#### *5.4.1 Lotions*

A lotion is a low- to medium-viscosity liquid formulation. A lotion can contain finely powdered substances that are insoluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In one embodiment, the lotion is in the form of an emulsion having a kinematic viscosity at 20°C of between 100 and 1000 $mm^2.s^{-1}$ (between 100 and 1000 cSt). The fluidity of lotions permits rapid and uniform application over a wide surface area. Lotions are typically intended to dry on the skin leaving a thin coat of their medicinal components on the skin's surface.

*5.4.2 Creams*

A cream is a viscous liquid or semi-solid emulsion of either the oil-in-water or water-in-oil type. Creams may contain emulsifying agents and/or other stabilizing agents. The oil-in-water cream bases are water-washable, and contain an oil phase, an emulsifier, and an aqueous phase. The oil phase, also called the 'internal' phase, is preferably comprised of petrolatum and a fatty alcohol such as cetyl or stearyl alcohol. The aqueous phase usually, although not necessarily, exceeds the oil phase in volume, and preferably contains a humectant. The emulsifier in a cream formulation is generally preferably a nonionic, anionic, cationic, or amphoteric surfactant. For the purposes of this invention a cream has a kinematic viscosity at 20°C of greater than 1000 $mm^2 \cdot s^{-1}$ (1000 cSt), typically in the range of 20,000-50,000 $mm^2 \cdot s^{-1}$ (20,000 to 50,000 cSt). Creams are oftentimes preferred over lotions and ointments as they are generally easier to spread and easier to remove.

The basic difference between a cream and a lotion is the viscosity, which is dependent on the amount/use of various oils and the percentage of water used to prepare the formulations. Creams are typically thicker than lotions and often use more varied oils/butters, depending upon the desired effect upon the skin. In a cream formulation, the water-base percentage is about 60-75% and the oil-base is about 20-30% of the total, with the other percentages being the active compounds or agents, emulsifier agents, preservatives and additives for a total of 100%.

*5.4.3 Gels*

A gel is a semisolid system containing dispersions of small or large molecules in a liquid vehicle that is rendered semisolid by the action of a thickening agent or polymeric material dissolved or suspended in the liquid vehicle. The liquid may include a lipophilic component, an hydrophilic component or both. Some emulsions may be gels or otherwise include a gel component. Some gels, however, are not emulsions because they do not contain a homogenized blend of immiscible components. Suitable gelling agents include, but are not limited to, modified celluloses, such as alginate, carboxymethyl cellulose, poly-ethylene glycol, hydroxypropyl cellulose and hydroxyethyl cellulose; carbopol homopolymers and copolymers; and combinations thereof. Suitable solvents in the liquid vehicle include, but are not limited to, diglycol monoethyl ether; alkene glycols, such as propylene glycol; dimethyl isosorbide; alcohols, such as isopropyl alcohol and ethanol; or water and buffers. The solvents are typically selected for their ability to dissolve the active compounds. Other additives, which improve the skin feel and/or emolliency of the formulation, may also be incorporated. Examples of such additives include, but are not limited, isopropyl myristate, ethyl acetate, C12-C15 alkyl benzoates, mineral oil, squalane, cyclomethicone, capric/caprylic triglycerides, and combinations thereof.

*5.4.4 Ointments*

An ointment is a semisolid preparation containing an ointment base and one or more of the active compounds or agents. Ointment bases may be grouped in four classes: oleaginous bases; emulsifiable-bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin, lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. A glycerol/HEPES buffer system can be used. Preferred water-soluble ointment bases are prepared from polyethylene glycols.

*5.4.5 Hydrogels and hydrocolloids*

Hydrogels generally consist of high-molecular weight molecules that form a coherent matrix for enclosing smaller molecules and aqueous solutions. Hydrogels can be described as a two- component system of water and a three-dimensional network polymer. Examples of hydrogels include alginate, PEG, CMC, starch, pectin, gelatine, other natural gums and insoluble cross-linked polymers such as polyethylene oxide. Hydrocolloids are hydrophilic polymers, of vegetable, animal, microbial or synthetic origin, that generally contain many hydroxyl groups and may be polyelectrolytes. They are naturally present or added to control the functional properties of a material such as viscosity, including thickening and gelling, and water binding. They are advantageous for use as wound care devices because of their ability to absorb several times their weight in wound exudates. Examples of hydrocolloids include carbowax, vinyl polymers (such as polyvinyl alcohol, polyvinyl pyrrolidone, and polyvinylacetate), cellulose derivatives (such as ethyl cellulose, methyl cellulose, and carboxymethyl cellulose), and natural gums (such as guar, acacia, and pectins).

The pH of the formulation (e.g. lotion, cream, gel, ointment, hydrogel or hydrocolloid) according to the present invention is preferably between 4 and 8, more preferably between 5 and 7.5, more preferably between 7.3 and 7.7.

### *5.4.6 Adhesive patch*

The compounds or formulations described herein may be incorporated into an adhesive patch. The adhesive patch of the invention comprises a backing layer and an adhesive layer disposed on at least one side of the backing. The adhesive patch of the invention can be sterilised by appropriate methods such as but not limited to irradiation. When selecting a suitable sterilization method, it is advisable to test and confirm that the sterilization does not lead to degradation or other inactivation of the compound of Formula 1, Formula 2 or Formulae 2-1 to 2-5.

The backing layer is preferably one which is substantially impermeable to components of the adhesive layer, such as a drug and additives, and prevents the components from passing through the backing and evaporating off from the back to cause a decrease in content. Examples of such backings include films of polyurethanes, polyesters such as poly(ethylene terephthalate), nylons, Saran™, polyethylene, polypropylene, poly(vinyl chloride), ethylene/ethyl acrylate copolymers, polytetrafluoroethylene, Surlyn™, and metal foils and laminated films composed of two or more thereof.

The adhesive layer is not particularly limited and examples of suitable adhesives include those based on acrylic polymers; rubber-based adhesives such as styrene/diene/styrene block copolymers (e.g., styrene/isoprene/styrene block copolymers and styrene/butadiene/styrene block copolymers), polyisoprene, polyisobutylene, and polybutadiene; silicone type adhesives such as silicone rubbers, dimethylsiloxane-based polymers, and diphenylsiloxane-based polymers; vinyl ether type adhesives such as poly(vinyl methyl ether), poly(vinyl ethyl ether), and poly(vinyl isobutyl ether); vinyl ester type adhesives such as vinyl acetate/ethylene copolymers; and polyester type adhesives produced from a carboxylic acid ingredient such as dimethyl terephthalate, dimethyl isophthalate, or dimethyl phthalate and a polyhydric alcohol ingredient such as ethylene glycol. The adhesive layer may be either a cross-linked adhesive layer or an uncross-linked adhesive layer. Preferably, the adhesive layer is a pressure-sensitive adhesive layer. From the standpoint of adhesion to the skin, hydrophobic pressure-sensitive adhesives are preferred and pressure-sensitive adhesive layers containing no water are preferred.

A release liner may also be disposed on the pressure-sensitive adhesive layer to improve ease of handling such as, for example, protect the adhesive layer and provide additional support (e.g. stiffness) before the patch is applied to a wound. The release liner is usually discarded before the adhesive patch is applied to the skin. It is therefore preferred, from the standpoint of inhibiting the adhesive patch from giving an uncomfortable feeling during wear on the skin, that the backing should be softer than the release liner. The release liner is not particularly limited. Examples of the material thereof include materials which are known in this field. Suitable release liner materials may, for instance, include bases having a release layer of a plastic film or paper which surface is treated with a release agent such as a silicone, long-chain alkyl, fluorine, or molybdenum sulphide release agent; low-adhesive bases comprising fluorine-containing polymers; and low adhesive bases comprising non-polar polymers including olefinic resins such as polyethylenes and polypropylenes to improve release property from an adhesive layer. Specific examples thereof include films of plastics such as polyesters including poly(ethylene terephthalate), poly(vinyl chloride), poly(vinylidene chloride), various acrylic and methacrylic polymers, polystyrene, polycarbonates, polyimides, cellulose acetate (acetate), regenerated cellulose (cellophane), and celluloid and laminated films composed of wood-free paper, glassine paper, or the like and a polyolefin. From the standpoints of safety, profitability, and drug migration prevention, it is preferred to use a polyester film. A preferred option is a fluorinated siliconized material.

The thickness of the release liner is generally 10 - 200 $\mu$m, preferably 25 - 100 $\mu$m. It is preferred to use a release liner having maximum peel force of less than 0.5 N/25mm when measured at a 180° peeling angle.

Drug-containing adhesive patches are well known and examples of how to prepare them can be found, for example, in WO 2013/005760 A1, EP 2332585 A1, EP 2570122 A1, EP 2702984 A1, WO 2012/014589 A1, WO 2009/124763 A2, WO 98/21578 A1, and the further references cited therein. Compounds according to Formula 1, Formula 2 or Formulae 2-1 to 2-5 can be employed in the patch in the skin-contact adhesive or in one or more additional layers in the patch (e.g. in a drug reservoir). Suitable drug-delivery patches include gelled or liquid reservoirs, such as in US 4,834,979, so-called "reservoir" patches; patches containing matrix reservoirs attached to the skin by an adjacent adhesive layer, such as in US 6,004,578, so-called "matrix" patches; and patches containing PSA reservoirs, such as in US 6,365, 178, US 6,024,976, US 4,751, 087 and US 6,149,935, so-called "drug-in-adhesive" patches.

In some embodiments, the drug reservoir can be provided in the form of a matrix layer containing drug, the matrix layer being adhered to the skin-contact adhesive of the patch. Such a matrix can be an adhesive layer and can include any of the adhesives described above. Alternatively, the matrix layer can be non-adhesive or weakly adhesive and rely upon a surrounding rim of skin-contact adhesive to secure the patch in place and keep the drug reservoir in contact with the skin surface.

Another topical administration form according to the present invention is a dressing with an absorbing patch in the middle (standard plaster type) where the compound according to Formula 1, Formula 2 or Formulae 2-1 to 2-5 is provided in the absorbing patch or on the surface in a resorbable matrix. In the first case the material will be into the absorbing material and be released by either exudate or skin moisture. If needed a secondary separating membrane could be used to ensure the correct water transport. In the last embodiment the compounds are embedded in a resorbable

material such as but not limited to PLA, alginate, or CMC that will release the compound upon contact with moisture. This film can have several architectures such as but not limited to a film, a porous film, a mesh, a fabric but it should allow the proper liquid transport. Underneath this film another potential absorbing material can be layered in order to capture the liquid such as blood, exudate, or sweat.

In some embodiments, the drug reservoir can be provided within the skin-contact adhesive of the patch. The drug can be mixed with the skin-contact adhesive prior to forming the patch or it may be applied to the skin-contact adhesive of the patch in a separate process step. Examples of suitable methods for applying drug to an adhesive layer may be found in US 2003/054025 and US 5,688,523.

Commercially available materials that can be modified to deliver compounds according to Formula (1) in the form of an adhesive patch include hydrocolloid dressing materials (e.g. DuoActive (Convatec), Comphile (Coloplast), Tegasorb (3M), Absocure (Nitto Medical Corporation)), alginate dressing materials (e.g. Cartstat (Convatec), Sorbsan (Alcare Co., Ltd), Algodam (Medion Inc.), Kurabio AG (Kuraray Co., Ltd) ), a hydrogen dressing materials (e.g. Jelleypalm (Taketora), New Jail (Johnson & Johnson), Intrasite (Smith & Nephew), Granugel (Convatec), Clearsite (Nippon Sigmax Co., Ltd)), polyurethane dressing materials (e.g. Tegaderm (3M), Opsite Wound (Smith & Nephew), IV3000 (Smith & Nephew), Bioclusive (Johnson & Johnson)), hydropolymer dressing materials (e.g. Tieral (Johnson & Johnson)), and hydrofiber dressing materials.

By administering the compounds according to Formula 1, Formula 2 or Formulae 2-1 to 2-5 by means of an adhesive patch, one can ensure a more uniform application of the compound as compared to alternative topical formulations. Furthermore, the adhesive patch serves to protect the wound while it is healing. Preferably, the active compounds of the invention are formulated in the adhesive patch in an amount suitable for release of 0.1 to 100,000 $mg/m^2$ or preferably 1 to 10,000 $mg/m^2$, more preferably 10 to 5,000 $mg/m^2$ and most preferably 20 to 2,500 $mg/m^2$. Depending on the release rate of the chosen formulation, the total amount needs to be adjusted according to the following formula:

$$\text{Amount in formulation } [\text{mg/mm}^2] = \text{amount to be released } [\text{mg/mm}^2] * (100/\text{RR } [\%])$$

Wherein RR is the release rate as determined according to the following experimental protocol: Attach the back of the test piece of 2.5 cm x 2.5 cm to the bottom of a petri dish by applying a suitable double coated adhesive. Place a 7 mL volume of medium, i.e. phosphate-buffered saline (specifically: 0.01 M phosphate buffer, 0.0027 M potassium chloride and 0.137 M sodium chloride, pH 7.4, at 25 °C), in the petri dish and seal with a top lid. Shake gently at 37 °C with a rate of 70 rpm. Samples are taken at sampling times of 1, 4, 8, 24 and 72 hours. At each sampling time, the medium is replaced by fresh medium. Determine the amount of substance in the medium of each sample by LC/MS. Release rate is calculated according to the following formulae:

$$\text{Release amount (RA)after } (n)\text{th time sampling } [\text{mg/m}^2] = \sum_{k=1}^{n} \left\{ \frac{\text{amount to be released in } (k)\text{th time } [\text{mg/ml}] * \text{volume of medium } [\text{ml}]}{\text{area of test species } [\text{m}^2]} \right\}$$

$$\text{Release rate } [\%] = (\text{RA } [\text{mg/m}^2] / \text{Substance amount in test piece } [\text{mg/m}^2]) \times 100\%$$

*5.4.7 Suture*

Sutures are well known in the art and are often used in surgical procedures for holding cut tissue surfaces in apposition for a period of time sufficient for healing. Non-absorbable sutures, e.g. sutures made from non-bioabsorbable materials such as polyolefins, nylon, cotton, and the like, are generally removed from the tissue after a period of time. Absorbable sutures, e.g. those fabricated from bioabsorbable materials such as polymers of lactide and glycolide, collagen, and the like, are gradually degraded and absorbed by the body, and do not require subsequent removal.

In a most preferred embodiment the suture is made from a biodegradable polymer as this allows more of the active agent to be released into the wound. Suitable biodegradable, biocompatible polymers include polyhydroxyacids such as poly(lactic acid), poly(glycolic acid), and poly(lactic acid-co-glycolic acids); polyhydroxyalkanoates such as poly-3-hydroxybutyrate or poly-4-hydroxybutyrate; polycaprolactones; poly(orthoesters); polyanhydrides; poly(phosphazenes); poly(hydroxyalkanoates); poly(lactide-co-caprolactones); polycarbonates such as tyrosine polycarbonates; polyamides (including synthetic and natural polyamides), polypeptides, and poly(amino acids); polyesteramides; polyesters; poly(dioxanones); poly(alkylene alkylates); hydrophobic polyethers; polyurethanes; polyetheresters; polyacetals; polycyanoacrylates; polyacrylates; polymethylmethacrylates; polysiloxanes; poly(oxyethylene)/poly(oxypropylene) copolymers; polyketals; polyphosphates; polyhydroxyvalerates; polyalkylene oxalates; polyalkylene succinates; poly(maleic acids), as well as copolymers thereof.

Preferable polymers include poly(lactide-coglycolide) (PLGA), polyglycolic acid (PGA), polylactic acid (PLA), polycaprolactone (PCL), polydioxanone (PDO), polyorthoester, polyanhydride, polyamino acid, polyhydroxybutyric acid, polycaprolactone, polyalkylcarbonate, ethyl cellulose, chitosan, starch, guargum, gelatin, collagen or a combination thereof. Exemplary polymeric materials for use in the invention that are already available in the market place are: Dexon II: homopolymer of glycolide, multifilament (Braun Surgical GmbH bzw. United States Surgical Corporation, North Haven, Conn.); Vicryl: copolymer of glycolide and lactide, multifilament (Ethicon, Inc., Sommerville, N.J.); copolymer of glycolide and lactide, multifilament (United States Surgical Corporation, North Haven, Conn.); PDS: homopolymer of p-dioxanone, monofilament (Ethicon, Inc., Sommerville, N.J.); Maxon: monofilament (United States Surgical Corporation, North Haven, Conn.); Biosyn: copolymer of p-dioxanone, trimethylencarbonate and glycolide, monofilament; (United States Surgical Corporation, North Haven, Conn.); Monocryl: block copolymer of glycolide and ε-caprolactone, monofilament (Ethicon, Inc., Sommerville, N.J.); and Bondek: homopolymer of diglycolide (Deknatel). Preferably, the polymer is PLGA.

The suture may contain one or more hydrophilic polymers. The hydrophilic polymer can be, for example, a poly(alkylene glycol), a polysaccharide, poly(vinyl alcohol), polypyrrolidone, a polyoxyethylene block copolymer (PLURONIC®) or a copolymers thereof. In preferred embodiments, the one or more hydrophilic polymers are, or are composed of, polyethylene glycol (PEG).

The polymer preferably has a molecular weight of about 150 Da to 1 MDa, more preferably about 500 Da to 500,000 Da and more preferably 10,000 to 100,000 Da. In certain embodiments, the biodegradable, biocompatible polymers has a molecular weight of between about 1 kDa and about 200kDa, more preferably between about 50kDa and about 150 kDa.

Methods of preparing sutures are well known in the art. For example, US 8,353,931 B2 describes a method of forming a barbed surgical suture from a degradable material. An absorbable suture made of a PGA polymer can be found in US 4,621,638. US 8,303,625 B2 describes a method for preparing sutures from degradable, thermoplastic polymers, which are able to change their shape after an increase in temperature. US 7,070,610 B2 describes methods of preparing monofilament sutures.

The compound of Formula 1, Formula 2 or Formulae 2-1 to 2-5 may be incorporated into the material forming the suture. Alternatively, it may also be incorporated into the suture by using a hollow suture, wherein the hollow core is filled with the compound of Formula 1, Formula 2 or Formulae 2-1 to 2-5 and thus acts as a reservoir.

The compound according to Formula 1, Formula 2 or Formulae 2-1 to 2-5 may be present in an amount of 0.1 to 40 wt.%, based on the total weight of the polymer and compound, in the suture material. Where more than one compound according to Formula 1, Formula 2 or Formulae 2-1 to 2-5 is present the amount is based on the sum amount of compounds according to these formulae. Preferably, the compound according to Formula 1, Formula 2 or Formulae 2-1 to 2-5 is present in an amount of 0.5 to 30 wt.%, more preferably 1 to 25 wt.%, and even more preferably 2 to 20 wt.%. Admixing of the compound(s) to the polymer can be done with the molten polymer or in solution.

### *5.4.8 Other topical administration forms*

According to one embodiment of the present invention, the compound of Formula 1, Formula 2 or Formulae 2-1 to 2-5 may be administered via a carrier that is coated with a suitable matrix having incorporated therein the compound of Formula 1, Formula 2 or Formulae 2-1 to 2-5. For instance, this may be vaseline or oily substance coated mesh, wherein the compound of Formula 1, Formula 2 or Formulae 2-1 to 2-5 is incorporated into the vaseline or oily substance matrix.

Suitable carrier materials according to this embodiment may thus include gauze or mesh materials made from cellulosic fibres, synthetic polymer fibres or any other material used for wound dressings. Such materials may be woven or non-woven.

The matrix material to be used according to this embodiment may be selected from any material (i.e. compound or composition) that is malleable or highly viscous but not solid at room temperature. As noted above, the group of suitable matrix materials includes vaseline, oily substances such as paraffin, hydrocolloids (e.g. microgranules of polysaccharides or gelatin covered by a polyurethane film), hydrogels (e.g. gels of hydrophilic polymers such as starch, agar or polyurethane and a high water content (typically above 35wt.%)), hydro fibres (e.g. carboxymethyl cellulose sodium fibres), foams, alginates, hyaluronic acid materials and the like.

The compound of Formula 1, Formula 2 or Formulae 2-1 to 2-5 may be incorporated into the matrix in solid (dispersed) form or in liquid (dissolved) form, depending on the type of matrix material.

According to another embodiment of the invention, the above-mentioned carrier material may be impregnated with the compound of Formula 1, Formula 2 or Formulae 2-1 to 2-5.

According to another embodiment of the invention, the compound of Formula 1, Formula 2 or Formulae 2-1 to 2-5 can be incorporated into an adhesive composition such as a medical or surgical glue.

Another topical administration form for the treatment of wounds caused by wet shaving relies on the incorporation of the compound of Formula 1, Formula 2 or

Formulae 2-1 to 2-5 in some part of the razor cartridge, such as wound healing composition strip above the blades, as disclosed in US 5,692,302 and especially columns 49 and 50 of this document.

Also direct injection is a valid option for administration of the compound of Formula 1, Formula 2 or Formulae 2-1 to 2-5. In this case, the compound may be provided in the form of a solution or dispersion within a liquid matrix suitable for injection. The injection should be administered into the wound or into the tissue adjacent to the wound.

The dosage for such alternative administration forms should be the same as indicated above for adhesive patches. That is, depending on the selected administration form, a dosage should be chosen, which allows effective administration of compound of Formula 1, Formula 2 or Formulae 2-1 to 2-5 in an amount of 0.1 to 100,000 mg/$m^2$ or preferably 1 to 10,000 mg/$m^2$, more preferably 10 to 1,000 mg/$m^2$ and even more preferably 50 to 500 mg/$m^2$. The actual dosage in the administration form must be adjusted as explained above, taking the release rate of the dosage form into account.

### *5.5 Excipients and other additives*

When formulated for topical administration the formulation may contain one or more excipients, diluents, carriers, additives and combinations thereof that may be combined with the active compounds to enhance, facilitate or enable the production, storage, administration, delivery or effectiveness of the active compounds. The carrier medium should be dermatologically acceptable, preferably designed to reduce or avoid undue toxicity, incompatibility, instability, allergic response and the like when applied to a wound or the skin.

The CTFA International Cosmetic Ingredient Dictionary and Handbook (2004 and 2008) describes a wide variety of non-limiting ingredients that can be used in the formulations of the present invention. Examples of these ingredient classes include: one or more skin penetration enhancers (which may be surfactants, alcohols, esters, glycols or the like or any other suitable penetration enhancer), emulsifiers, moisturizers (including, e.g., emollients, humectants, film formers, occlusive agents, and agents that affect the natural moisturisation mechanisms of the skin), skin conditioning agents (e.g., aloe extracts, allantoin, bisabolol, ceramides, dimethicone, hyaluronic acid), anti-irritants (e.g. steroids and non-steroidal anti-inflammatories), anti-microbial agents (e.g. antibiotics, silver), fragrances (artificial and natural), dyes and colour ingredients (e.g., Blue 1, Blue 1 Lake, Red 40, titanium dioxide, D&C blue no. 4, D&C green no. 5, D&C orange no. 4, D&C red no. 17, D&C red no. 33, D&C violet no. 2, D&C yellow no. 10, and D&C yellow no. 11), adsorbents, lubricants, solvents, water-repellents, UV absorbers (physical and chemical absorbers such as paraminobenzoic acid ("PABA") and corresponding PABA derivatives, titanium dioxide, zinc oxide, etc.), essential oils, vitamins (e.g. A, B, C, D, E, and K), trace metals (e.g. zinc, calcium and selenium), botanical extracts (e.g. *Aloe vera*, chamomile, cucumber extract, *Ginkgo biloba*, ginseng, and rosemary), antioxidants (e.g., BHT, BHA and tocopherol), chelating agents (e.g., disodium EDTA and tetrasodium EDTA), preservatives (e.g., methylparaben and propylparaben), pH adjusters (e.g., sodium hydroxide and citric acid), absorbents (e.g., aluminum starch octenylsuccinate, kaolin, corn starch, oat starch, cyclodextrin, talcum, and zeolite), skin bleaching and lightening agents (e.g., hydroquinone and niacinamide lactate), humectants (e.g., urea and mannitol), exfoliants, waterproofing agents (e.g., magnesium/aluminum hydroxide stearate), and dipotassium glycyrrhizate). Other additional ingredients include surfactants (which may be cationic, non-ionic, anionic or polymeric), clays, anti-foaming agents, spreading agents, barriers, solubilising agents for the therapeutic agent and the like. Other exemplary compounds for different classes mentioned above can be found in the CTFA International Cosmetic Ingredient Dictionary and Handbook. For instance, Section 3 of Volume 3 (2004) lists various compounds in terms of their function (pages 2177 to 2299).

Examples of skin penetrating agents that can be used in the invention include alcohols such as dodecanol and oleyl alcohol; amines, such as isopropyl amine, diisopropyl amine, triethyl amine, triethanol amine, diisopropanolamine and ethylene diamine; carboxylic acids, such as erucic acid, oleic acid, linoleic acid, linolenic acid, and bahemic acid; esters, such as dibutyl sebacate, dibutyl phthalate, butyl benzoate, ethyl caprate and dimethyl isosorbide; and others, such as azone, N-methyl pyrollidone, bile salts and urea.

Examples of emulsifiers that can be used in the invention include, but are not limited to, methyl glucose sesquistearate, PEG-20 methyl glucoside sesquistearate, steareth-21, polyethylene glycol 20 sorbitan monostearate, polyethylene glycol 60 sorbitan monostearate, polyethylene glycol 80 sorbitan monostearate, steareth-20, ceteth-20, PEG-100 stearate, sodium stearoyl sarcosinate, hydrogenated lecithin, sodium cocoylglyceryl sulfate, sodium stearyl sulfate, sodium stearoyl lactylate, PEG-20 glyceryl monostearate, sucrose monostearate, sucrose polystearates, polyglyceryl 10 stearate, polyglyceryl 10 myristate, steareth 10, DEA oleth 3 phosphate, DEA oleth 10 phosphate, PPG-5 ceteth 10

phosphate sodium salt, PPG-5 Ceteth 10 phosphate potassium salt, steareth-2, PEG-5 soya sterol oil, PEG-10 soya sterol oil, diethanolamine cetyl phosphate, sorbitan monostearate, diethylenglycol monostearate, glyceryl monostearate, and mixtures thereof.

Examples of emollients that can be used in the invention include, but are not limited to, almond oil, caprylic/capric triglycerides, castor oil, ceratonia extract, ceteareth-20, ceteareth-30, cetearyl alcohol, ceteth 20, cetostearyl alcohol, cetyl alcohol, cetyl stearyl alcohol, cetyl esters wax, cottonseed oil, cocoa butter, cyclomethicone, diisopropyl adipate, ethylene glycol palmitostearate, glycerin, glyceryl monooleate, glyceryl monostearate, glyceryl stearate, isopropyl myristate, isopropyl palmitate, lanolin, lanolin alcohol, hydrogenated lanolin, lecithin, liquid paraffins, linoleic acid, light mineral oil, medium-chain triglycerides, mineral oil, oleic acid, white petrolatum, polyethylene glycol, polyoxyethylene glycol fatty alcohol ethers, polyoxypropylene 15-stearyl ether, propylene glycol stearate, squalane, steareth-2 or - 100, stearic acid, soybean oil, starch, stearyl alcohol, sunflower oil, xylitol, urea and mixtures thereof.

Examples of humectants that can be used in the invention include, but are not limited to, propylene glycol, glycerin, butylene glycol, triacetin and mixtures thereof. Examples of diluents that can be used in the invention include but are not limited to, lactose, sugar, starches, modified starches, mannitol, inorganic salts, cellulose derivatives (e.g. microcrystalline cellulose, cellulose), calcium sulfate, xylitol, lactitol and the like and mixtures thereof.

Examples of buffering agents that can be used in the invention include sodium hydroxide, potassium hydroxide, ammonium hydroxide and mixtures thereof.

Examples of chelating agents that can be used in the invention include mild agents, such as, for example, ethylenediaminetetraacetic acid (EDTA), disodium edetate and EDTA derivatives, and mixtures thereof.

Examples of preservatives include that can be used in the invention phenoxyethanol, parabens (such as methylparaben and propylparaben), propylene glycols, sorbates, urea derivatives (such as diazolindinyl urea), and mixtures thereof.

Any of the above excipients can be included in the adhesive patch of the invention.

### *5.6 Additional active agents*

The active compounds of Formula 1, Formula 2 or Formulae 2-1 to 2-5 suitable for practising the invention can be used in simultaneous or sequential combination with one or more other active agents ('additional active agent'). For example, when applied topically the active compound can be applied before, together with, or after an additional active agent that is normally delivered onto or through the skin for either a local or systemic effect. When applied simultaneously, the active compound of the invention according to Formula 1, Formula 2 or Formulae 2-1 to 2-5 and the additional active agent may be present in the same topical formulation (e.g. in the form of a lotion, cream, gel, or ointment).

The additional active agent includes antibiotics, analgesics, anesthetics, anti-inflammatory agents (e.g., steroidal compounds such as dexamethasone, betamethasone, prednisone, prednisolone, triamcinolone, hydrocortisone, alclometasone, amcinonide, diflorasone, etc. as well as non-steroidal anti-inflammatories), anti-itch and irritation-reducing compounds (e.g., antihistamines such as diphenhydramine and psoriasis treatments), antimicrobial agents, antiseptic agents (e.g., povidone-iodine, methylbenzethonium chloride, etc.), immunomodulating agents, vitamins and the like.

Suitable antibiotics include cilastatin, clavulanic acid, folinic acid, probenecid, pyridoxine, sulbactam, dapsone, ethambutol, isoniazid, pyrazinamide, rifampin, streptomycin, capreomycin, ethionamide, para aminosalicylic acid, cycloserine, ciprofloxacin, nalidixic acid, norfloxacin, ofloxacin, imipenam, meropenem, cilistatin, cefadroxil, cefazolin, cephalexin, cephalothin, cefaclor, cefamandole, cefonicid, cefoxitin, cefuroxine, cefoperazone, cefotaxime, ceftazidime, ceftizoxime, ceftriaxone, moxalactam, cefepine, bacitracin, vancomycin, aztreonam, amoxicillin, clavulanic acid, benzathine, penicillin g, penicillin v, ampicillin, carbenicillin. indamyl, carbenicillin, mezlocillin, piperacillin, ticarcillin, cloxacillin, dicloxacillin, floxacillin, methicillin, nafcillin, oxacillin, colistmethate, polymixin b, trimethoprim, cotrimoxazole, mafenide, sulfadiazine, sodium sulfacetamide, sulfacytine, sulfadiazine, sulfamethoxazole, sulfapyridine, sulfasalazine, sulfisoxazole, chloramphenicol, clindamycin, spectinomycin, azithromycin, clarithromycin, erythromycin, erythromycin estolate, spiramycin, chlortetracycline, demeclocycline, doxycycline, minocycline, oxytetracycline, amikacin, kanamycin, neomycin, streptomycin, tobramycin, nitrofurantoin, griseofulvin, potassium iodide, fluconazole, itraconazole, ketoconazole, miconazole, clotrimazole, amphotericin b, nystatin, niclosamide, nifurtimox, piperazine, praziquantel, pyrantel pamoate, ascariasis, pinworm, thiabendazole, amodiaquine, chloroquine, hydroxychloroquine, mefloquine, primaquine, pyrimethamine, quinidine gluconate, fansidar, diloxanide furoate, melarsoprol, nifurtimox, paromomycin, pentamidine, sodium stibogluconate, suramin, metronidazole, foscarnet, 3-deoxythmidin-2-ene, dideoxycytosine, dideoxyinosine, lamivudine, azidothymidine, indinavir, ritonavir, saquinavir, acyclovir, idoxuridine, ribavirin, vidarabine, amantidine, rinantidine, pharmaceutically acceptable salts thereof, and combinations thereof. Specifically, the antibiotic can be at least one of arnphomycin, apramycin, avilamycin, azithromycin, bacitracin, bactiracin zinc, clarithromycin, clindamycin, clindamycin hydrochloride, clindamycin palmitate hydrochloride, clindamycin phosphate, dirithromycin, erythromycin, erythromycin acistrate, erthromycin estolate, erthryomycin ethlylsuccinate, erthryomycin gluceptate, erythromycin lactobionate, erthromycin propionate, erthromycin stearate, fosfomycin, fosfomycin tromethamine, josamycin, kitasamycin, lexithromycin, lincomycin, limcomycin hydrochloride, metronidazole hydrochloride, metronidazole phosphate, mirincamycin hydrochloride, paldimycin, paulomycin, pirlimycin hydrochloride, ranimycin, relomycin, roxithromycin, spectinomycin hydrochloride, spiramycin, stallimycin hydrochloride, tobramycin, vancomycin, vancomycin hydrochloride, zorbamycin, mupirocin, mupirocin calcium, and parachlorophenol.

Suitable analgesics include nonsteroidal anti-inflammatory drugs (NSAIDs) (e.g., salicylates, propionic acid derivatives, acetic acid derivatives, enolic acid derivatives, fenamic acid derivatives, COX-2 inhibitors, sulphonanilides, etc.) and opiates (e.g., morphine, codeine, thebaine, papaverine, etc.). Exemplary analgesics include acetaminophen, alfentanil hydrochloride, aminobenzoate potassium, aminobenzoate sodium, anidoxime, anileridine, anileridine hydrochloride, anilopam hydrochloride, anirolac, antipyrine, aspirin, benoxaprofen, benzydamine hydrochloride, bicifadine hydrochloride, brifentanil hydrochloride, bromadoline maleate, bromfenac sodium, buprenorphine hydrochloride, butacetin, butixirate, butorphanol, butorphanol tartrate, carbamazepine, carbaspirin calcium, carbiphene hydrochloride, carfentanil citrate, ciprefadol succinate, ciramadol, ciramadol hydrochloride, clonixeril, clonixin, codeine, codeine phosphate, codeine sulfate, conorphone hydrochloride, cyclazocine, dexoxadrol hydrochloride, dexpemedolac, dezocine, diflunisal, dihydrocodeine bitartrate, dimefadane, dipyrone, doxpicomine hydrochloride, drinidene, enadoline hydrochloride, epirizole, ergotamine tartrate, ethoxazene hydrochloride, etofenamate, eugenol, fenoprofen, fenoprofen calcium, fentanyl citrate, floctafenine, flufenisal, flunixin, flunixin meglumine, flupirtine maleate, fluproquazone, fluradoline hydrochloride, flurbiprofen, hydromorphone hydrochloride, ibufenac, indoprofen, ketazocine, ketorfanol, ketorolac tromethamine, letimide hydrochloride, levomethadyl acetate, levomethadyl acetate hydrochloride, levonantradol hydrochloride, levorphanol tartrate, lofemizole hydrochloride, lofentanil oxalate, lorcinadol, lornoxicam, magnesium salicylate, mefenamic acid, menabitan hydrochloride, meperidine hydrochloride, meptazinol hydrochloride, methadone hydrochloride, methadyl acetate, methopholine, methotrimeprazine, metkephamid acetate, mimbane hydrochloride, mirfentanil hydrochloride, molinazone, morphine sulfate, moxazocine, nabitan hydrochloride, nalbuphine hydrochloride, nalmexone hydrochloride, namoxyrate, nantradol hydrochloride, naproxen, naproxen sodium, naproxol, nefopam hydrochloride, nexeridine hydrochloride, noracymethadol hydrochloride, ocfentanil hydrochloride, octazamide, olvanil, oxetorone fumarate, oxycodone, oxycodone hydrochloride, oxycodone terephthalate, oxymorphone hydrochloride, pemedolac, pentamorphone, pentazocine, pentazocine hydrochloride, pentazocine lactate, phenazopyridine hydrochloride, phenyramidol hydrochloride, picenadol hydrochloride, pinadoline, pirfenidone, piroxicam olamine, pravadoline maleate, prodilidine hydrochloride, profadol hydrochloride, propiram fumarate, propoxyphene hydrochloride, propoxyphene napsylate, proxazole, proxazole citrate, proxorphan tartrate, pyrroliphene hydrochloride, remifentanil hydrochloride, salcolex, salicylamide, salicylate meglumine, salsalate, sodium salicylate, spiradoline mesylate, sufentanil, sufentanil citrate, talmetacin, talniflumate, talosalate, tazadolene succinate, tebufelone, tetrydamine, tifurac sodium, tilidine hydrochloride, tiopinac, tonazocine mesylate, tramadol hydrochloride, trefentanil hydrochloride, trolamine, veradoline hydrochloride, verilopam hydrochloride, volazocine, xorphanol mesylate, xylazine hydrochloride, zomepirac sodium, and zucapsaicin.

Suitable anesthetics include aliflurane, articaine, benoxinate hydrochloride, benzocaine, biphenamine hydrochloride, bupivacaine hydrochloride, butamben, butamben picrate, chloroprocaine hydrochloride, cocaine, cocaine hydrochloride, cyclopropane, desflurane, dexivacaine, diamocaine cyclamate, dibucaine, dibucaine hydrochloride, dyclonine hydrochloride, enflurane, ether, ethyl chloride, etidocaine, etoxadrol hydrochloride, euprocin hydrochloride, fluroxene, halothane, isobutamben, isoflurane, ketamine hydrochloride, levobupivacaine, levoxadrol hydrochloride, lidocaine, lidocaine hydrochloride, mepivacaine hydrochloride, methohexital sodium, methoxyflurane, midazolam hydrochloride, midazolam maleate, minaxolone, norflurane, octodrine, oxethazaine, phencyclidine hydrochloride, pramoxine hydrochloride, prilocaine hydrochloride, procaine hydrochloride, propanidid, proparacaine hydrochloride, propofol, propoxycaine hydrochloride, pyrrocaine, risocaine, rodocaine, roflurane, ropivacaine, salicyl alcohol, sevoflurane, teflurane, tetracaine, tetracaine hydrochloride, thiamylal, thiamylal sodium, thiopental sodium, tiletamine hydrochloride, and zolamine hydrochloride.

Suitable immunomodulating agents, include azathioprine, 6- mercaptopurine, cyclosporin, methotrexate, interferon $\alpha$ IIb, autologous granulocyte macrophagecolony stimulating factor, APC 8015 (Provenge), cancer vaccines, antisense oligonucleotides, bacillus of Calmette-Guerin (BCG), and the like.

Other additional active agents include isosorbide, isomannide, isoidide, isosorbide mono- and diesters having C1-C12 alkyl groups (e.g. Polysorb ID 37 available from Roquette, France), salicylic acid, cholesterol benzoate, retinyl acetate, ammonium bituminosulfonate (ichthammol), chloorhexidine, and sucrose octasulphate.

Any of the above additional active agents can be included in the adhesive patch or suture of the invention.

## 6. Amount of active compound and dosing regimen

### *6.1 Amount of active compound*

The topical formulations of the present invention typically contain 0.1 to 10,000 $\mu$M, preferably from 0.3 to 3000 $\mu$M, more preferably 1 to 1000 $\mu$M of the active compound according to Formula 1, Formula 2 or Formulae 2-1 to 2-5.

Alternatively or additionally, the topical formulations of the invention contain from 0.0001 to 100 wt.%, preferably 0.0005 to 10 wt.%, more preferably 0.001 to 2 wt.%, even more preferably 0.01 to 0.1 wt.% of the active compound according to Formula 1, Formula 2 or Formulae 2-1 to 2-5. If a mixture of two or more of the active compounds of Formula 1, Formula 2 or Formulae 2-1 to 2-5 is present, the above ranges apply to the sum of the weight contents of the active compounds that are present.

The active compound should be used in a therapeutically effective amount. A "therapeutically effective amount" means the amount of the active compound (or mixture thereof) that, when administered to a patient for treating a wound or reducing scarring, is sufficient to induce a medically beneficial effect. The therapeutically effective amount may vary depending on the active compound, the specific nature of the wound and the age, weight or other characteristic of the patient to be treated.

When considered as a function of area, it is preferable to apply the compound evenly over a surface to be treated so as to arrive at an amount of 1 to 10,000 $mg/m^2$, preferably 10 to 1,000 $mg/m^2$ and more preferably 50 to 500 $mg/m^2$.

### *6.2 Dosing regimen*

The dosage may be delivered by a single administration or by multiple applications as needed to achieve the most effective results. Dosing can continue for as long as is medically indicated, which will depend on the severity of the wound. The dosage may also be delivered in a controlled-release manner such as with an adhesive patch or suture.

It is preferable to treat a wound as quickly as possible in view of increased skin permeability immediately after wounding. Low molecular weight (< 500 Da) and moderately lipophilic agents (logP 1-3) can surmount the horny barrier layer and gain access to viable epidermis, dermis and blood vessels to a low, yet relevant extent (Bätz et al. 2012).

When treating acute wounds, it is preferable that the compounds according to Formula 1, Formula 2 or Formulae 2-1 to 2-5 (or a formulation comprising these compounds) is applied to the wound (or closed wound) within 1 day of the wound being formed. It is more preferable to apply the compounds within 6 hours of wound formation, even more preferable within 1 hour of wound formation. If necessary, further applications of the compounds according to Formula 1, Formula 2 or Formulae 2-1 to 2-5 (or a formulation comprising these compounds) can be applied. For example, the compounds or formulations can be applied as one, two, three or more separate applications. In one embodiment, the compounds according to Formula 1, Formula 2 or Formulae 2-1 to 2-5 (or a formulation comprising these compounds) are applied to the wound on a daily basis for 2, 3, 4, 5, 6, 7 or more days.

For wounds created during surgery and subsequently closed, the compounds according to Formula 1, Formula 2 or Formulae 2-1 to 2-5 (or a formulation comprising these compounds) are preferably applied to the open wound and again to the closed wound. The compounds or formulations can be applied a number of times to the closed wound. For example, the compounds or formulations can be applied as one, two, three or more separate applications. The compounds or formulations can be applied to the wound on a daily basis for 2, 3, 4, 5, 6, 7 or more days.

An alternative option is to apply the compounds according to Formula 1, Formula 2 or Formulae 2-1 to 2-5 (or a formulation comprising these compounds) to a wound by means of an adhesive patch, wound dressing or suture.

The present invention is now illustrated in greater detail by way of the following examples, but it should be understood that the present invention is not deemed to be limited thereto.

## 7. Examples

### 7.1 Preparation of compositions

Composition 1: Glycerol 50% v/v containing 20 mM HEPES and 150 mM NaCl.

Composition 2: Glycerol 50% v/v containing 20 mM HEPES, 150 mM NaCl and 0.1 μM Lipoxin A4.

Composition 3: Glycerol 50% v/v containing 20 mM HEPES, 150 mM NaCl and 1 μM Lipoxin A4.

Additional ingredients that are common in topical formulations have been kept to a minimum so as to examine the effect that a compound according to Formula (1) has on the reduction of scarring. Further topical formulations are described below.

### 7.2 Animal preparation and treatment

Mouse models have contributed significantly to an understanding of skin biology and disease and so are an appropriate model for testing the compounds and formulations of the invention. The mouse model used here can be considered one of the simplest and standard murine models for investigating scar formation and wound healing.

#### *7.2.1 Anaesthesia and surgery*

Mice were weighed and then anaesthetized with 5% isoflurane in air for induction and then administered 2.5-3% of isoflurane in air for the remainder of the procedure. The animals were administered Tramal (Tramadol) intraperitoneally for analgesia at a 0.1 mg/kg dose. Before surgery the back of the mice was shaved and disinfected with isobetadine soap, rinsed with water and then swabbed with iodium alcohol.

#### *7.2.2 Excisional model (wound left open):*

Four full-thickness skin wounds were performed by cutting the folded skin on the back of the mice with a 7.5 mm x 3 mm elliptical biopsy punch (Elliptical Excisional Instrument, Acuderm Inc.). The skin on the back was raised and folded at the level of the vertebral column by using the thumb and the index finger. The folded skin was then placed on a wood tongue depressor with the animal laying on one side and two wounds were performed through the folded skin by using an elliptical biopsy punch (Elliptical Excisional Instrument, Acuderm Inc.), resulting in four wounds on the back skin: two on the right of the vertebral column and two on the left (Figure 1). The epidermis, the dermis and the fat tissue in the wound was carefully removed without touching other tissues. The wounds were then immediately treated with topical application of Composition 1 ($T_v$ - vehicle), Composition 2 ($T_1$ - 1 μM Lipoxin A4), or Composition 3 ($T_2$ - 100 μM Lipoxin A4). The position of the wounds that were treated with active agent ($T_1$ or $T_2$) or treated with vehicle ($T_v$) was randomised. The wound site was then covered with OP-Film (Somed International, the Netherlands). The borders were then fixed with cyanoacrylate glue (Loctite).

#### *7.2.3 Post-operative care*

The mice were allowed to recover from the surgical preparation in a clean cage with fresh bedding and under a heating lamp. To avoid possible contamination the mice were placed in individual cages containing a cellulose bed that was changed every 24 hours. Single caging avoids other mice from detaching the adhesive patches.

#### *7.2.4 Post-operative treatment*

The treatment was repeated at the day 1, 2, 3 and 4 post-surgery for a total of 5 treatments in the first 5 days of healing. At each time point the mice were briefly anaesthetized with isoflurane and a new dose of ointment was injected through the film onto the wound, after which the animal was left to recover. At day 5 all films were removed.

#### *7.2.5 Tissue harvest*

After 14 days the mice were sacrificed under isoflurane anaesthesia by cervical dislocation to harvest the regenerated tissue. Skin samples were taken. The back of the mouse was carefully shaved paying careful attention to not touch the wound area. Three deep cuts in the back skin were made using a surgical blade to harvest two skin strips containing the four residual wounds (two wounds per strip). A scissors was used to harvest a square of skin containing the residual wound.

The skin wounds were split into two parts perpendicular to the direction of the incision. One part was imbedded in OCT and frozen on dry ice and the last one fixed in 4% paraformaldehyde for embedding in paraffin.

The segment for cryosectioning was prepared as follows. The mold was partially filled with the OCT medium, on top of which the sample was placed. Hereafter the remainder of the mold was filled with OCT. Care was taken not to have air bubbles close to the samples and a mark was made to identify the side of the wound. The sample was frozen by placing the mold on dry ice. The samples were stored at -20°C until shipment.

The other part of the section was fixed for further processing. The sample was placed between two pieces of paper on which a marking was made identifying the wound side of the sample. The sample was then placed in a cassette and a polyurethane sponge was placed on top. The cassette was closed and placed in a 4% ice-cold paraformaldehyde solution for 24 hrs after which they were transferred to 70% ethanol solution and paraffin-embedded for sectioning.

### 7.3 Measuring the extent of scarring

The extent of scarring was measured in accordance with the procedure set out by Khorasani et al. (2011).

H&E staining photographs were captured on an an automated Imager Z2 microcope (Zeiss Microscopy) at 20× magnification. Image analysis was performed using the ZEN image acquisition and analysis software (Zeiss).

Dermal thickness was defined as the distance from the epidermal-dermal junction down to the panniculus carnosus visualized by H&E stain. Using Zen, dermal thickness measurements were obtained on 40× light microscope images by drawing a line normal to the average orientation of the epidermal-dermal and dermalsubcutaneous tissue demarcations. Four dermal thickness measurements were taken per sample-two adjacent to the wound site at 50 μm on either side, and two at a farther distance of 700 μm on either side of the wound.

Fibrotic scar tissue was outlined using the freeform outline tool in Zen to produce a pixel-based area measurement that could then be converted to square micrometers. Like the dermal thickness measurements, scar area measurements were performed extended to the panniculus carnosus. Scar size is determined by the Scar Index, which is calculated by dividing the scar area (A, in $\mu m^2$) by the corresponding average dermal thickness ($T_{avg}$, in μm):

$$\text{Scar Index} = A/T_{avg}$$

Collagen content and organisation were assessed as published by De Visscher et al. (2007, 2008). In short, sections are stained with picrosirius red and then imaged at 20x. The imaging is done with a mosaic of overlays of bright-field and polarised light images. A semi-automatic analysis tool was developed allowing assessment of the surfaces covered by collagen and organised collagen. From this the collagen content and organisation can be measured according to the following method.

The total area (At) of the region of interest (ROI) and the area of the red stained material within that area (Ar) on the bright-field image. On the polarized light image, the area of the red illuminated bundles (Ab) is measured. The collagen density and organized collagen are calculated by the following formulae:

$$\text{(1) Collagen density: } (Ar/At) \times 100$$

$$\text{(2) Organized collagen: } (Ab/Ar) \times 100$$

Both are expressed as a percentage: collagen density as a percentage of the total area and organized collagen as a percentage of the collagen area. As can be seen from

Fig. 5, a wound treated with a compound according to the present invention exhibited reduced collagen content and organisation.

### 7.4 Results

Histological analysis was performed and the scar index measured (Table 1) according to the protocol described above. A statistically significant decrease (1-way ANOVA with Dunnet's comparison of pooled Lipoxin A4-treated group to Vehicle-treated group, p=0.008) in the scar index was observed when wounds were treated with Lipoxin A4 (Figure 3: Vehicle 1478 μm ± 334 μm; Pooled Lipoxin A4 treatment: 748 μm ±217 μm). A dose-dependent reduction of scar index was observed for the Lipoxin A4-treated group (Figure 4: Vehicle 1478 μm ± 334 μm; 1 μM Lipoxin A4 treatment: 905 μm ± 146 μm; 100 μM Lipoxin A4 treatment: 511 μm ±130 μm).

Collagen content was measured by image analysis in order to quantify the amount of collagen in the wounds (Table 1). Lower collagen content was observed in wounds treated with vehicle (5%) than wounds treated with Lipoxin A4. Collagen organisation showed a similar trend with increased values upon administration of Lipoxin A4.

Table 1:

| Composition | Average of Collagen Content in the wound (%) | Average of Collagen organisation in the wound (%) |
|---|---|---|
| | | |
| Composition 1 (Vehicle) | 73 | 21 |

(continued)

| Composition | Average of Collagen Content in the wound (%) | Average of Collagen organisation in the wound (%) |
|---|---|---|
| Composition 2 | 64 | 8 |

### 7.5 Further topical formulations

The following topical formulations can also be used in methods for reducing scarring according to the invention.

**Cream formulation (ingredient and wt.%)**

| | |
|---|---|
| Lipoxin A4 | 0.15% |
| Stearic acid | 6% |
| Stearyl alcohol | 4% |
| Cetyl alcohol | 2.5% |
| Glycerin | 12% |
| Sodium lauryl sulfate | 1% |
| Propylparaben | 0.3% |
| Disodium EDTA | 0.055% |
| Distilled water | QS |

**Ointment formulation (ingredient and weight)**

| | |
|---|---|
| Lipoxin A4 | 1 g |
| Cholesterol | 32 g |
| Stearyl Alcohol | 33 g |
| White Wax | 75 g |
| White Petrolatum | 859 g |

**Gel formulation (ingredient and wt.%)**

| | |
|---|---|
| Lipoxin A4 | 0.1% |
| Methylparaben | 0.10% |
| Propylparaben | 0.05% |
| Carbomer 934P NF | 1.5% |
| Sodium Hydroxide | QS pH 7 |
| Purified Water USP | QS 100% |

**Lotion formulation (ingredient and weight)**

| | |
|---|---|
| Lipoxin A4 | 0.001 g |
| Castor oil | 4.5 g |
| Petrolatum | 1.2 g |
| PEG fatty alcohol ether | 2.7 g |

(continued)

| | |
|---|---|
| Butylated hydroxytoluene (BHT) | 0.02 g |
| Propylene glycol | 13 g |
| Purified Water USP | QS 100 g |

**Hydrogel formulation (ingredient and wt.%)**

| | |
|---|---|
| Lipoxin A4 | 1% |
| Propylene Glycol | 13.5% |
| Triacetin (Glycerol Triacetate) | 5.0% |
| Ethanol | 17.6% |
| Lauryl Alcohol | 0.8% |
| Nonanol (Nonyl Alcohol) | 0.8% |
| Pure Water | 20.3% |
| Hydroxyethyl Cellulose (Mn: 250,000) | 6.0% |
| Polyvinyl Pyrrolidine (Collidon 90™) | 10.0% |
| 25% PVA Aqueous Solution (Degree of Polymerization: 500-2,000) | 25.0% |

The formulations above should not be considered as a limitation upon the formulations that can be used according to the invention but merely as being illustrative and representative thereof. Methods for preparing these formulations are known, or will be apparent, to those skilled in the art. See, e.g., "Remington: The Science and Practice of Pharmacy, 21st Ed., Chapter 39" and "Ansel's Pharmaceutical Dosage Forms and Drug Delivery Systems, 9th Ed., Section IV-10 and Section VI". Reference is also made to the CTFA International Cosmetic Ingredient Dictionary and Handbook (2004 and 2008) which describes a wide variety of ingredients (such as those above as well as possible replacements or additional ingredients) that can be used in the formulations of the present invention.

**8. Industrial applicability**

The present invention provides compounds and formulations that are useful for reducing the formation of scar tissue.

**9. Cited References**

Bätz, Franzisca Marie et al. 2012. "Esterase activity in excised and reconstructed human skin - Biotransformation of prednicarbate and the model dye fluorescein diacetate." European journal of pharmaceutics and biopharmaceutics : official journal of Arbeitsgemeinschaft fur Pharmazeutische Verfahrenstechnik e.V. Retrieved May 21, 2013 (http://www.ncbi.nlm.nih.gov/pubmed/23201050).

De Visscher, Geofrey et al. 2007. "In vivo cellularization of a cross-linked matrix by intraperitoneal implantation: a new tool in heart valve tissue engineering." European heart journal 28(11):1389-96.

De Visscher, Geofrey et al. 2008. "Functional and biomechanical evaluation of a completely recellularized stentless pulmonary bioprosthesis in sheep." The Journal of thoracic and cardiovascular surgery 135(2):395-404.

Khorasani, Hooman et al. 2011. "A quantitative approach to scar analysis." The American journal of pathology 178(2):621-8.

Singer, A J, and R A Clark. 1999. "Cutaneous wound healing." The New England journal of medicine 341(10):738-46.

**Claims**

1. A compound according to Formula 1, or a pharmaceutically acceptable salt or solvate thereof, for use in a method of reducing scar formation in a wound:

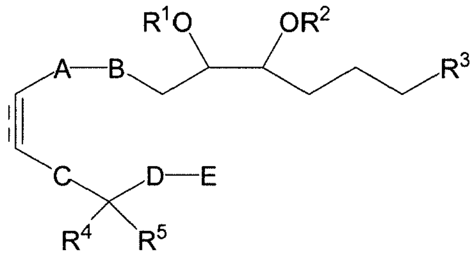

Formula 1

wherein

is a double bond or triple bond, preferably a double bond;
A, B and C are the same or different and selected from $CH_2CH_2$, *cis*-CH=CH, *trans*-CH=CH and C≡C;
$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;
$R^3$ is $CO_2R^{31}$, $CONR^{32}R^{33}$, $CH_2OR^{34}$, $CH_2NR^{35}R^{36}$, $CH_2N_3$, $CH_2$-X, $CH_2NO_2$, $CH_2SR^{37}$, $COSR^{38}$, or 2,3,4,5-tetrazol-1-yl, wherein:

$R^{31}$ is H or $C_{1-6}$ alkyl;
$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
$R^{34}$ is H, $C_{1-6}$ alkyl, or $C_{2-6}$ acyl;
$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
X is F, Cl, Br or I;
$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;

$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;
D is $(CR^6R^7)_q$ or $(CR^6R^7)_qO$, where q is 0-6; and $R^6$ and $R^7$ are the same or different and selected from H or $C_{1-6}$ alkyl;
E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

2. The compound for use according to claim 1, having the structure:

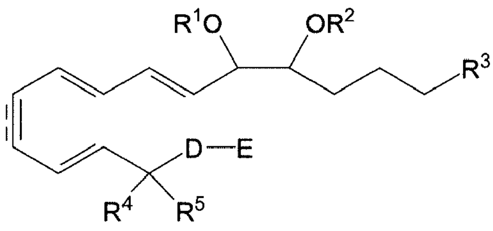

Formula 2

**3.** The compound for use according to claim 2, having the structure:

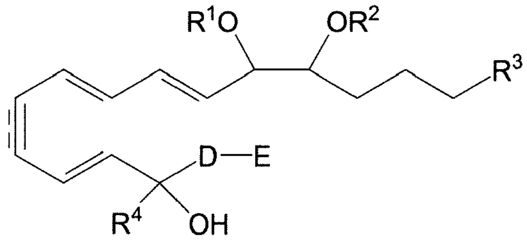

Formula 2-1

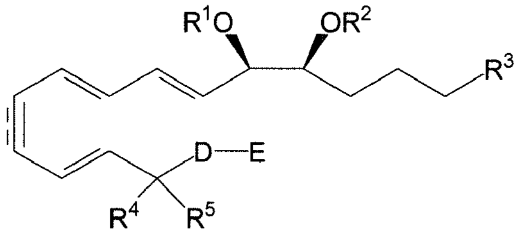

Formula 2-2

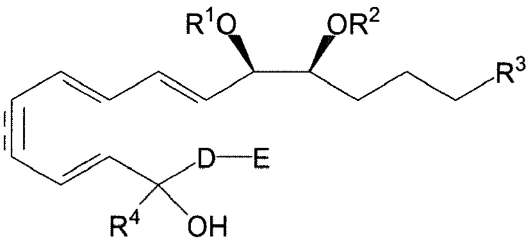

Formula 2-3

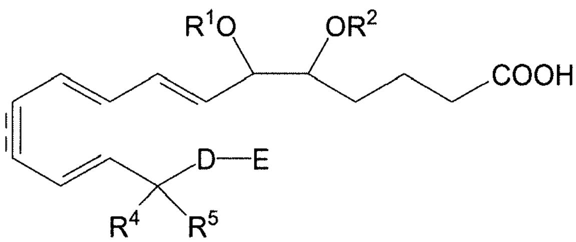

Formula 2-4

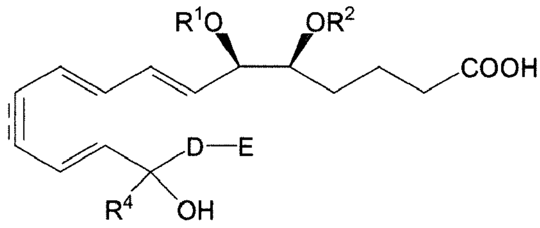

Formula 2-5

**4.** The compound for use according to any one of claims 1 to 3, wherein $R^1$ and $R^2$ are H.

**5.** The compound for use according to any one of claims 1 to 3, wherein $R^3$ is COOH.

**6.** The compound for use according to any one of claims 1 to 3, wherein $R^4$ is H or $CH_3$.

**7.** The compound for use according to any one of claims 1 to 3, wherein $R^5$ is OH.

**8.** The compound for use according to any one of claims 1 to 3, wherein
$R^7$ and $R^2$ are H, and $R^3$ is COOH; or
$R^1$ and $R^2$ are H, and $R^4$ is H or $CH_3$; or
$R^1$ and $R^2$ are H, and $R^5$ is OH; or
$R^3$ is COOH, and $R^4$ is H or $CH_3$; or
$R^3$ is COOH, and $R^5$ is OH; or
$R^4$ is H or $CH_3$, and $R^5$ is OH.

**9.** The compound for use according to any one of claims 1 to 3, wherein
$R^1$ and $R^2$ are H, $R^3$ is COOH, and $R^4$ is H or $CH_3$; or
$R^1$ and $R^2$ are H, $R^3$ is COOH, and $R^5$ is OH; or
$R^1$ and $R^2$ are H, $R^3$ is COOH, $R^4$ is H or $CH_3$, and $R^5$ is OH.

**10.** The compound for use according to any one of claims 1 to 9, wherein D is $-C_4H_8-$ or $-CH_2O-$, and/or wherein E is $-CH_3$, cyclohexyl or optionally substituted phenyl.

**11.** The compound for use according to any one of claims 1 to 10, wherein the method of reducing scar formation comprises the step of topically applying the compound to the wound bed of an open wound.

**12.** The compound for use according to any one of claims 1 to 10, wherein the method of reducing scar formation comprises the step of topically applying the compound to the area around a closed wound.

**13.** A topical formulation for use in a method of reducing scar formation in a wound, comprising a compound as defined in any one of claims 1 to 12.

**14.** An adhesive patch, an adhesive composition, or a suture comprising at least one compound according to Formula (1), or a pharmaceutically acceptable salt or solvate thereof:

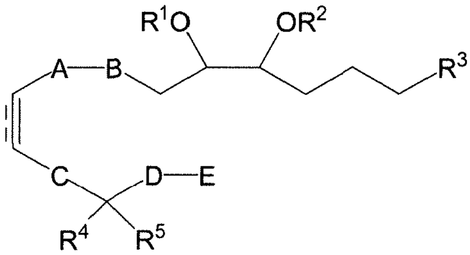

Formula 1

wherein

is a double bond or triple bond, preferably a double bond;
A, B and C are the same or different and selected from $CH_2CH_2$, *cis*-CH=CH, *trans*-CH=CH and C≡C;
$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;
$R^3$ is $CO_2R^{31}$, $CONR^{32}R^{33}$, $CH_2OR^{34}$, $CH_2NR^{35}R^{16}$, $CH_2N_3$, $CH_2$-X, $CH_2NO_2$, $CH_2SR^{37}$, $COSR^{38}$, or 2,3,4,5-tetrazol-1-yl, wherein:

$R^{31}$ is H or $C_{1-6}$ alkyl;
$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
$R^{34}$ is H, $C_{1-6}$ alkyl, or $C_{2-6}$ acyl;
$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
X is F, Cl, Br or I;
$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;

$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;
D is $(CR^6R^7)q$ or $(CR^6R^7)qO$, where q is 0-6; and $R^6$ and $R^7$ are the same or different and selected from H or $C_{1-6}$ alkyl;
E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

**15.** A wound dressing comprising a carrier that is coated with a matrix having incorporated therein the compound of Formula (1) or a pharmaceutically acceptable salt or solvate thereof or comprising a carrier that is impregnated with a compound of Formula (1) or a pharmaceutically acceptable salt or solvate thereof:

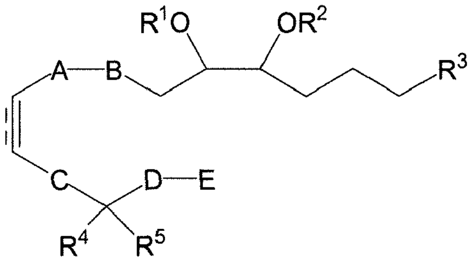

Formula 1

wherein

is a double bond or triple bond, preferably a double bond;
A, B and C are the same or different and selected from $CH_2CH_2$, *cis*-CH=CH, *trans*-CH=CH and C≡C;
$R^1$ and $R^2$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-6}$ acyl;
$R^3$ is $CO_2R^{31}$, $CONR^{32}R^{33}$, $CH_2OR^{34}$, $CH_2NR^{35}R^{36}$, $CH_2N_3$, $CH_2$-X, $CH_2NO_2$, $CH_2SR^{37}$, $COSR^{38}$, or 2,3,4,5-tetrazol-1-yl, wherein:

$R^{31}$ is H or $C_{1-6}$ alkyl;
$R^{32}$ and $R^{33}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
$R^{34}$ is H, $C_{1-6}$ alkyl, or $C_{2-6}$ acyl;
$R^{35}$ and $R^{36}$ are the same or different and selected from H, $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl, OH, or $C_{1-6}$ alkoxy;
X is F, Cl, Br or I;
$R^{37}$ and $R^{38}$ are H or a $C_{1-6}$ alkyl, $C_{2-6}$ alkenyl, $C_{2-6}$ alkynyl, $C_{4-8}$ cycloalkyl, $C_{4-8}$ cycloalkenyl, $C_{6-12}$ aralkyl, $C_{5-10}$ aryl;

$R^4$ and $R^5$ are the same or different and selected from H, $C_{1-6}$ alkyl, OH, $C_{1-6}$ alkoxy, $C_{1-6}$ alkoxy, $C_{2-6}$ alkenyloxy, $C_{2-6}$ alkynyloxy, $C_{4-8}$ cycloalkyloxy, $C_{4-8}$ cycloalkenyloxy, $C_{6-12}$ aralkyloxy, $C_{5-10}$ aryloxy, $C_{1-7}$ carbamate, and $C_{2-12}$ acyl, or together form a double bonded oxygen;
D is $(CR^6R^7)_q$ or $(CR^6R^7)$qO, where q is 0-6; and $R^6$ and $R^7$are the same or different and selected from H or $C_{1-6}$ alkyl;
E is $CH_3$, or a cyclohexyl or phenyl ring optionally substituted with one or more substituents selected from $C_{1-6}$ alkyl, halogen, trihalomethyl, $C_{2-6}$ acyl, hydroxy, thiol, or amino group.

FIGURES

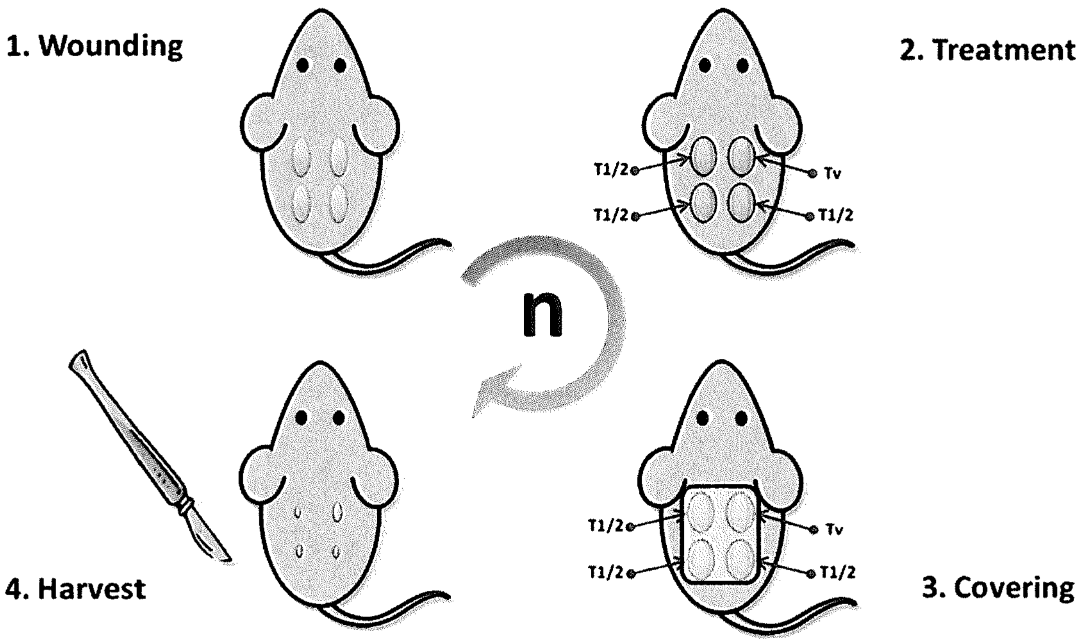


FIG. 1

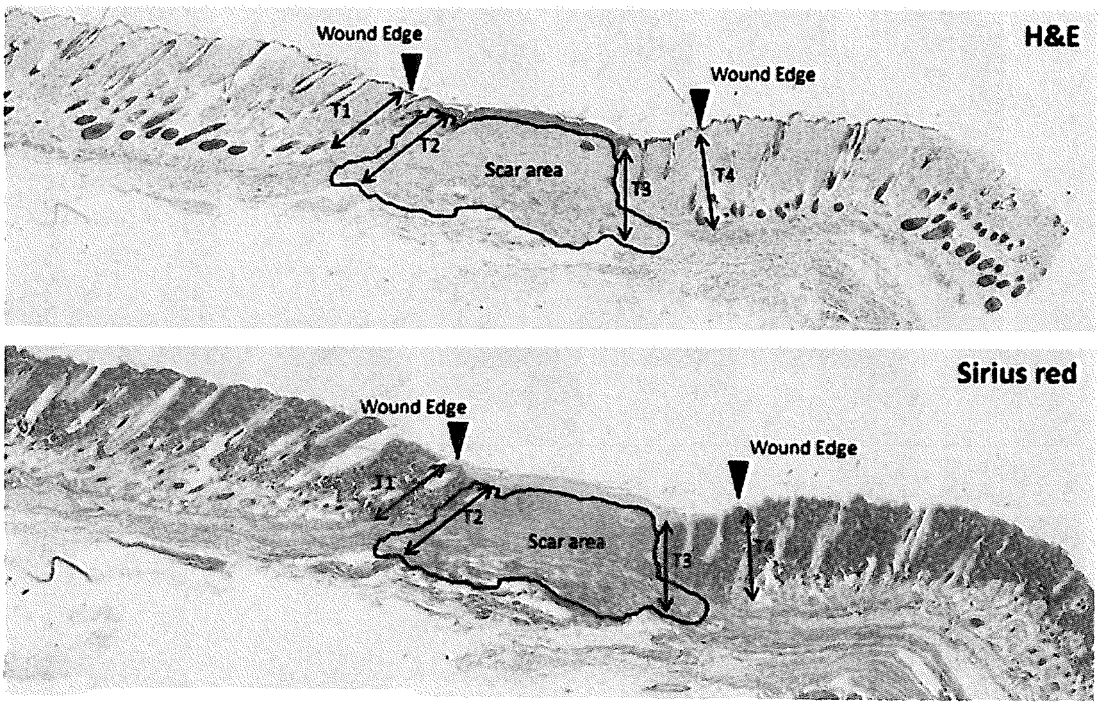
H&E
Wound Edge
Wound Edge
T1
T2
T3
T4
Scar area
Sirius red
Wound Edge
Wound Edge
T1
T2
T3
T4
Scar area

FIG. 2

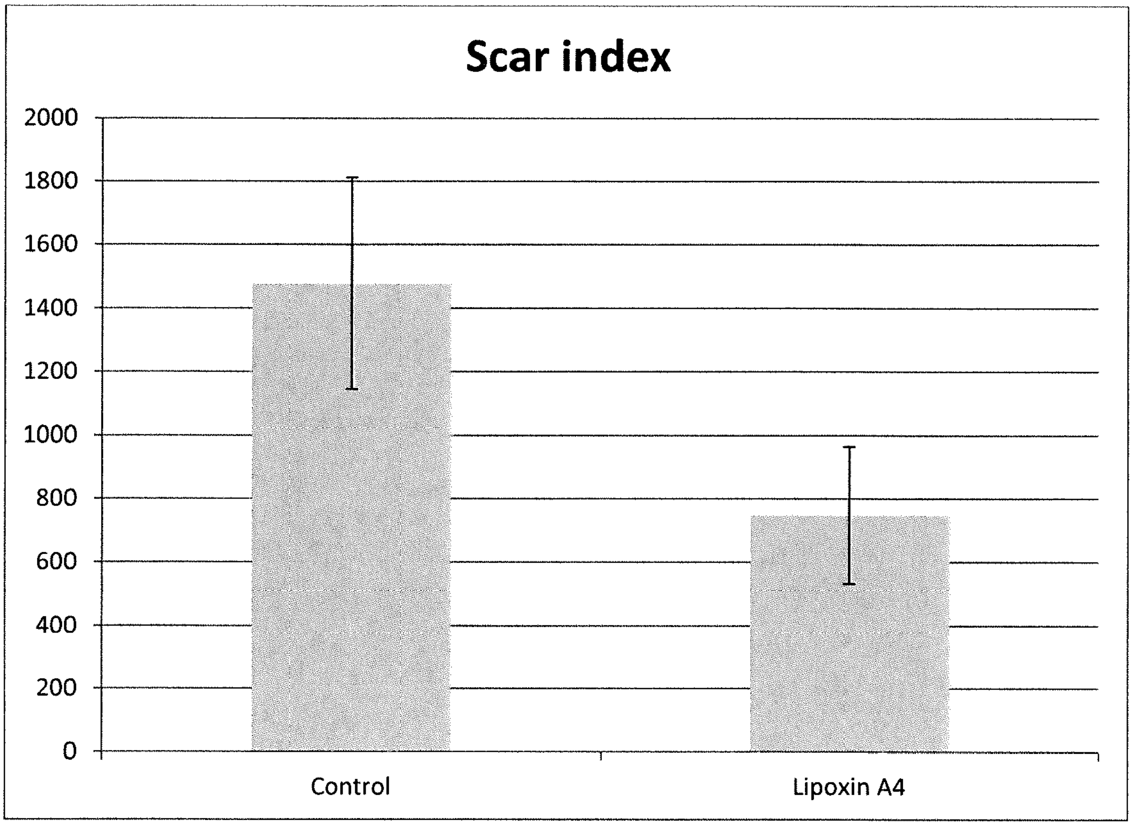
Scar index
2000
1800
1600
1400
1200
1000
800
600
400
200
0
Control
Lipoxin A4

FIG. 3

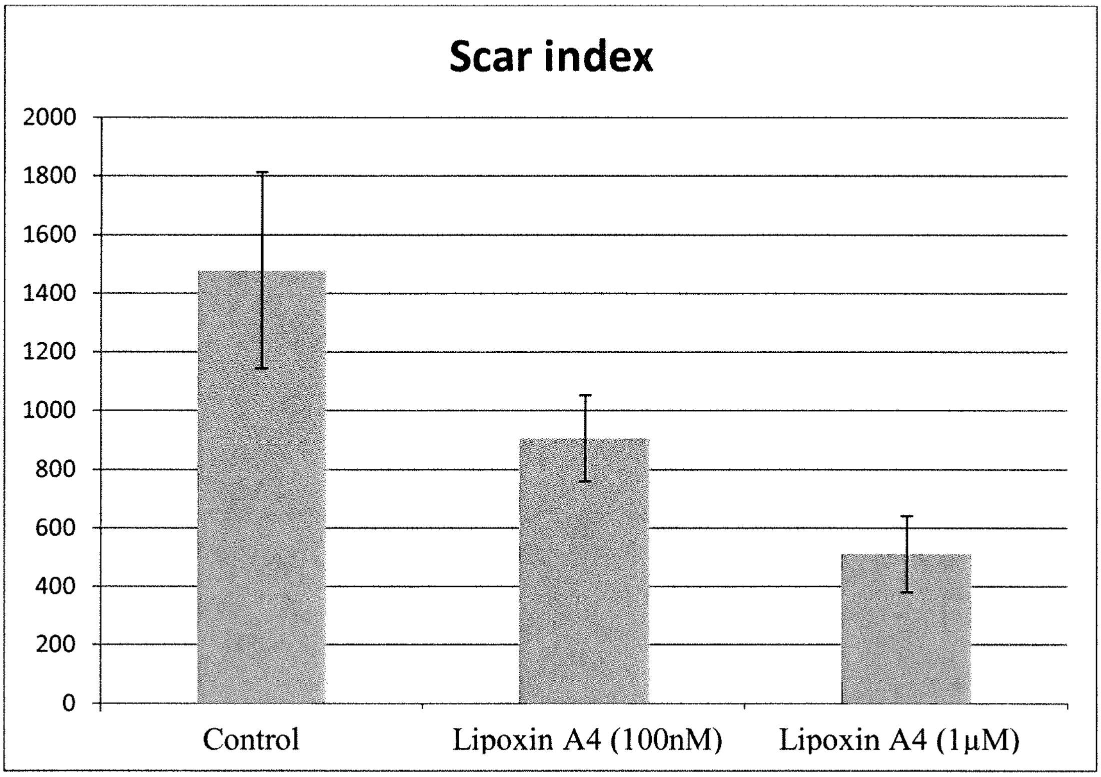
Scar index
2000
1800
1600
1400
1200
1000
800
600
400
200
0
Control
Lipoxin A4 (100nM)
Lipoxin A4 (1µM)

FIG. 4

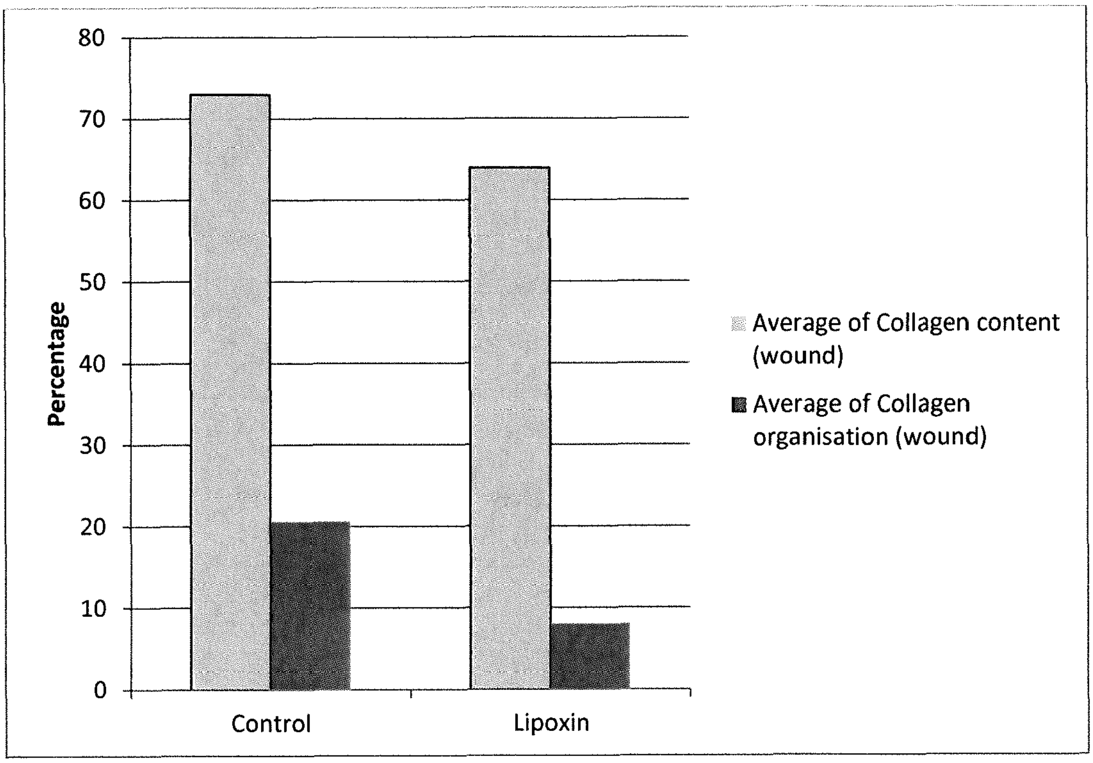
80
70
60
50
40
30
20
10
0
Percentage
Control
Lipoxin
Average of Collagen content (wound)
Average of Collagen organisation (wound)

FIG. 5

Europäisches Patentamt
European Patent Office
Office européen des brevets

# EUROPEAN SEARCH REPORT

Application Number
EP 14 19 3497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; 20 December 2013 (2013-12-20), ZHOU, XIAOYAN ET AL: "The effect and mechanism of lipoxin A4 suppressing scar formation", XP002738727, retrieved from STN Database accession no. 2013:1961845 * abstract * | 1-15 | INV. A61K31/202 A61K9/70 A61P17/02 |
| X | WO 02/070068 A2 (BRIGHAM & WOMENS HOSPITAL [US]) 12 September 2002 (2002-09-12) * abstract * * page 11, line 24 - line 28 * * page 16, line 8 - line 16 * * page 22, line 27 - line 32; claims 1-4 * | 1-14 | |
| X | US 2008/161275 A1 (GJORSTRUP PER [US]) 3 July 2008 (2008-07-03) * abstract * * page 2, paragraph 12 * * page 13, paragraph 193 - paragraph 321; claim 1 * | 1-14 | TECHNICAL FIELDS SEARCHED (IPC) A61K |
| X | BITEMAN BENJAMIN ET AL: "Interdependence of lipoxin A4 and heme-oxygenase in counter-regulating inflammation during corneal wound healing.", FASEB JOURNAL : OFFICIAL PUBLICATION OF THE FEDERATION OF AMERICAN SOCIETIES FOR EXPERIMENTAL BIOLOGY JUL 2007, vol. 21, no. 9, July 2007 (2007-07), pages 2257-2266, XP0055184638, ISSN: 1530-6860 * the whole document * | 1-11,13 | |

-/--

1

The present search report has been drawn up for all claims

| Place of search | Date of completion of the search | Examiner |
|---|---|---|
| The Hague | 21 April 2015 | Hoff, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 3497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAKAZU AZUCENA ET AL: "Lipoxin A4inhibits platelet-activating factor inflammatory response and stimulates corneal wound healing of injuries that compromise the stroma", EXPERIMENTAL EYE RESEARCH, vol. 103, 2012, pages 9-16, XP028942926, ISSN: 0014-4835, DOI: 10.1016/J.EXER.2012.07.008 * the whole document * ----- | 1-11,13 | |
| X | US 2005/203184 A1 (PETASIS NICOS A [US]) 15 September 2005 (2005-09-15) * page 1, paragraph 7 * * page 3, paragraph 24 * * page 9, paragraph 107 * * page 14, paragraph 157 - paragraph 158 * * page 14, paragraph 163; claims 2,3 * ----- | 14,15 | |

TECHNICAL FIELDS SEARCHED (IPC)

The present search report has been drawn up for all claims

1

| Place of search | Date of completion of the search | Examiner |
|---|---|---|
| The Hague | 21 April 2015 | Hoff, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 3497

This annex lists the patent family members relating to the patent documents cited in the above-mentioned European search report. The members are as contained in the European Patent Office EDP file on
The European Patent Office is in no way liable for these particulars which are merely given for the purpose of information.

21-04-2015

| Patent document cited in search report | Publication date | Patent family member(s) | Publication date |
|---|---|---|---|
| WO 02070068 A2 | 12-09-2002 | AT 412448 T | 15-11-2008 |
| | | AU 2002252175 A1 | 19-09-2002 |
| | | CY 1108670 T1 | 09-04-2014 |
| | | DK 1406698 T3 | 09-02-2009 |
| | | EP 1406698 A2 | 14-04-2004 |
| | | EP 2221089 A1 | 25-08-2010 |
| | | ES 2316553 T3 | 16-04-2009 |
| | | JP 5302856 B2 | 02-10-2013 |
| | | JP 2005508282 A | 31-03-2005 |
| | | JP 2010059183 A | 18-03-2010 |
| | | JP 2012255025 A | 27-12-2012 |
| | | PT 1406698 E | 12-01-2009 |
| | | US 2002193431 A1 | 19-12-2002 |
| | | US 2004053998 A1 | 18-03-2004 |
| | | US 2006009521 A1 | 12-01-2006 |
| | | US 2008064749 A1 | 13-03-2008 |
| | | WO 02070068 A2 | 12-09-2002 |
| US 2008161275 A1 | 03-07-2008 | US 2008161275 A1 | 03-07-2008 |
| | | WO 2008070129 A2 | 12-06-2008 |
| US 2005203184 A1 | 15-09-2005 | US 2005203184 A1 | 15-09-2005 |
| | | US 2006270734 A1 | 30-11-2006 |
| | | US 2010152290 A1 | 17-06-2010 |
| | | US 2012142772 A1 | 07-06-2012 |
| | | US 2015073047 A1 | 12-03-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013005760 A1
- EP 2332585 A1
- EP 2570122 A1
- EP 2702984 A1
- WO 2012014589 A1
- WO 2009124763 A2
- WO 9821578 A1
- US 4834979 A
- US 6004578 A
- US 6365 A
- US 178 A
- US 6024976 A
- US 4751 A
- US 087 A
- US 6149935 A
- US 2003054025 A
- US 5688523 A
- US 8353931 B2
- US 4621638 A
- US 8303625 B2
- US 7070610 B2
- US 5692302 A


### Non-patent literature cited in the description


- CTFA International Cosmetic Ingredient Dictionary and Handbook. 2004, vol. 3, 2177-2299
- Remington: The Science and Practice of Pharmacy
- Ansel's Pharmaceutical Dosage Forms and Drug Delivery Systems
- CTFA International Cosmetic Ingredient Dictionary and Handbook. 2004
- **BÄTZ, FRANZISCA MARIE et al.** Esterase activity in excised and reconstructed human skin - Biotransformation of prednicarbate and the model dye fluorescein diacetate. *European journal of pharmaceutics and biopharmaceutics : official journal of Arbeitsgemeinschaft fur Pharmazeutische Verfahrenstechnik e.V,* 2012, http://www.ncbi.nlm.nih.gov/pubmed/23201050
- **DE VISSCHER, GEOFREY et al.** In vivo cellularization of a cross-linked matrix by intraperitoneal implantation: a new tool in heart valve tissue engineering. *European heart journal,* 2007, vol. 28 (11), 1389-96
- **DE VISSCHER, GEOFREY et al.** Functional and biomechanical evaluation of a completely recellularized stentless pulmonary bioprosthesis in sheep. *The Journal of thoracic and cardiovascular surgery,* 2008, vol. 135 (2), 395-404
- **KHORASANI, HOOMAN et al.** A quantitative approach to scar analysis. *The American journal of pathology,* 2011, vol. 178 (2), 621-8
- **SINGER, A J ; R A CLARK.** Cutaneous wound healing. *The New England journal of medicine,* 1999, vol. 341 (10), 738-46